(19) Europäisches Patentamt
European Patent Office
Office européen des brevets

(11) EP 3 156 058 B1

(12) EUROPEAN PATENT SPECIFICATION

(45) Date of publication and mention of the grant of the patent:
**27.05.2020 Bulletin 2020/22**

(21) Application number: **15809254.4**

(22) Date of filing: **15.06.2015**

(51) Int Cl.:
***A61K 31/704*** *(2006.01)* ***A61K 36/36*** *(2006.01)*
***A61P 35/00*** *(2006.01)*

(86) International application number:
**PCT/CN2015/081493**

(87) International publication number:
**WO 2015/192758 (23.12.2015 Gazette 2015/51)**

(54) **ANTI-TUMOR PHARMACEUTICAL APPLICATION OF PENTACYCLIC TRITERPENE SAPONIN COMPOUNDS OF SZECHUAN MELANDIUM ROOT**

ANTITUMORALE PHARMAZEUTISCHE ANWENDUNG VON PENTACYCLISCHEN TRITERPENSAPONINVERBINDUNGEN DER SZECHUAN-MELANDIUM-WURZEL

APPLICATION PHARMACEUTIQUE ANTI-TUMORALE DE COMPOSÉS DE SAPONINE DE TRITERPÈNE PENTACYCLIQUE DE RACINE DE SZECHUAN MELANDIUM

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.06.2014 CN 201410267578**

(43) Date of publication of application:
**19.04.2017 Bulletin 2017/16**

(73) Proprietors:
- **Wang, Xueyong**
  **Beijing 100029 (CN)**
- **Zhao, Baosheng**
  **Beijing 100029 (CN)**

(72) Inventors:
- **Wang, Xueyong**
  **Beijing 100029 (CN)**
- **Zhao, Baosheng**
  **Beijing 100029 (CN)**

(74) Representative: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**

(56) References cited:
**CN-A- 103 599 117 CN-A- 104 013 636**

- **"Isolation and Characterization of Chemical Constituents from the Roots of Silene viscidula Franch.", ASIAN JOURNAL OF CHEMISTRY, vol. 25, no. 18, 1 January 2013 (2013-01-01), XP055248131, IN ISSN: 0970-7077, DOI: 10.14233/ajchem.2013.15288**
- **WEI XU ET AL: "Pentacyclic Triterpenoid Saponins from Silene viscidula", HELVETICA CHIMICA ACTA, vol. 93, no. 10, 1 October 2010 (2010-10-01), pages 2007-2014, XP055248134, CH ISSN: 0018-019X, DOI: 10.1002/hlca.201000016**
- **Jing Zhao ET AL: "New triterpenoid saponins from the roots of Sinocrassula asclepiadea", Chem. Pharm. Bull., 1 January 2004 (2004-01-01), pages 230-237, XP055248075, Retrieved from the Internet: URL:https://www.jstage.jst.go.jp/article/cpb/52/2/52_2_230/_pdf [retrieved on 2016-02-05]**
- **Hong Bai ET AL: "A major triterpenoid saponin from Gypsophila oldhamiana", Chemistry & biodiversity, 1 May 2007 (2007-05-01), page 955, XP055433760, Switzerland Retrieved from the Internet: URL:http://onlinelibrary.wiley.com/store/10.1002/cbdv.200790085/asset/955_ftp.pdf?v=1&t=jb1yfrth&s=285eeac89060027d04c8cccd25cbf6ae83d7d292**
- **FU, HONGZHENG ET AL.: 'Silenorubicosides A-D, Triterpenoid Saponins from Silene rubicunda' JOURNAL OF NATURAL PRODUCTS vol. 68, 05 November 2005, pages 754 - 758, XP055244234**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 156 058 B1

## Description

### Field of the Invention

The present invention relates to a novel use of pentacyclic trieterpenoid saponins from *silene viscidula* and their compositions for use as anti-tumor agents, and the application of these compounds in the preparation of anti-tumor drugs.

### Background

Malignant tumor has been a major disease urgent need to be tackled. At present, the toxicity and adverse reactions of anti-tumor drugs have been increasingly paid more attention. It is the focus and key point to seek anti-tumor drugs with high efficiency and low toxicity. Medical plants as a great treasure of human beings are still ideal material resources for anti-tumor drugs screening and show a good application prospect. For example, paclitaxel, camptothecin, ginsenoside and a number of natural compounds with strong anti-tumor activity were screened out from plants. China is a large country with a long history of using herbal medicine and gradually formed a unique theoretical system of traditional Chinese medicine (TCM) in the process of Chinese people fighting with diseases. Under the guidance of TCM theory, many medicinal plants are used and played an important role in treating difficult miscellaneous diseases like cancers. In addition, China's rich natural herbal resources and clinical experiences of ethnic and folk medicine in the treatment of cancer have laid a good foundation for screening anti-tumor drugs with high efficiency and low toxicity. Our research firstly discovered that compounds of pentacyclic trieterpenoid saponins from *silene viscidula* showed strong anti-tumor activity both in vitro and in vivo.

*Silene viscidula* (Called *Wacao* in Chinese) is a species of plant belongs to the family Caryophyllaceae, the root of which is known as *'Wacao'* by the hmong people in China. According to theory of TCM, *Wacao* has the effects of analgesia, hemostasia, clearing heat and diuretic, and is often clinically used to treat traumatic injury, rheumatic pain, bronchitis, and urinary tract infection. Currently, researches of *Silene viscidula* are mainly concentrated on its chemical compositions, but barely on pharmacology. The main chemical compositions of *Silene viscidula* are saponins, proteins, organic acids, polysaccharides, and cyclic peptides, etc. Our former work had successfully separated, purified and identified a number of pentacyclic trieterpenoid saponins from *Silene viscidula* which laid the good foundation for screening and evaluating their anti-tumor activities.

"Isolation and Characterization of Chemical Constituents from the Roots of Silene viscidula Franch." ASIAN JOURNAL OF CHEMISTRY, vol. 25, no. 18, 1 January 2013, discloses eight compounds isolated and characterized by alcohol extraction method from the roots of *Silene viscidula* Franch.

Wei Xu et al., "Pentacyclic Triterpenoid Saponins from Silene viscidula", HELVETICA CHIMICA ACTA, vol. 93, no. 10, 1 October 2010, pages 2007-2014 describes other triterpenoid saponins isolated from *Silene viscidula.* Zhao, J., Nakamura, N., Hattori, M., Yang, X.W., Komatsu, K. and Qiu, M.H., 2004. New triterpenoid saponins from the roots of Sinocrassula asclepiadea. Chemical and pharmaceutical bulletin, 52(2), pp.230-237, discloses five new triterpenoid monodesmosides and six bisdesmosides isolated from the roots of *Sinocrassula asclepiadea* Franch.

Bai, H., Zhong, Y., Xie, Y.Y., Wang, Y.S., Liu, L., Zhou, L., Wang, J., Mu, Y.L. and Zuo, C.X., 2007. A major triterpenoid saponin from Gypsophila oldhamiana. Chemistry & biodiversity, 4(5), pp.955-960, discloses a new saponin, gypoldoside isolated from the roots of *Gypsophila oldhamiana.*

Summary of the Invention

The invention relates to the compounds of general formula I or their salts, compositions containing the formula I or their salts for use in the treatment of tumor. The embodiments of the present invention are reflected in independent claims 1, 6 and 9. The preferred embodiments of the present invention are reflected in dependent claims 2-5, 7, 8, and 10-13.

The object of the invention is realized by the following technical scheme:

The invention discloses the compounds of general formula I or their salts, compositions containing the formula I or their salts for use in the treatment of tumor.

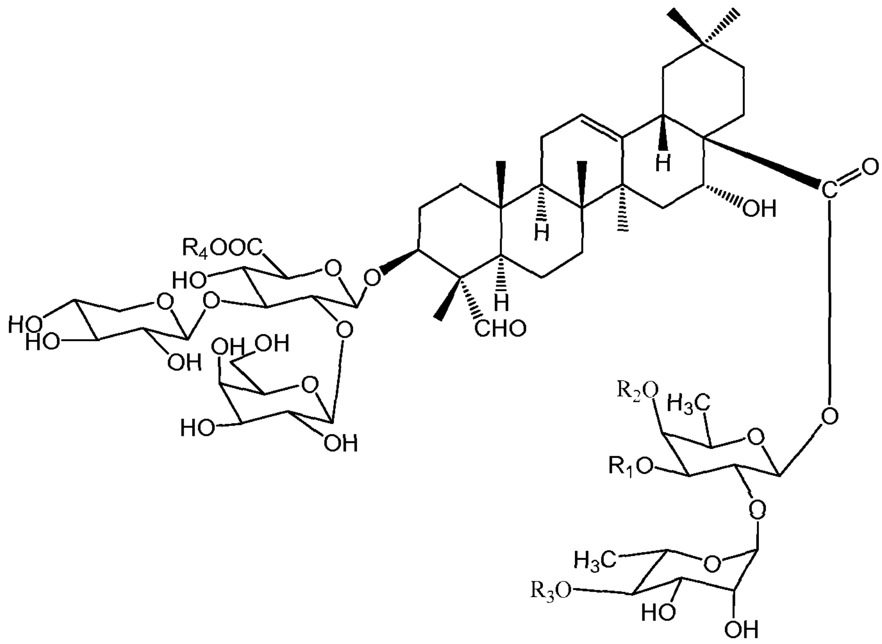

Formula I

In the general formula I:

$R_1$= Ac,
$R_2$= (E) -MC, (Z)-MC,
$R_3$=H,
$R_4$=H, $CH_3$, or $CH_2CH_2CH_2CH_3$;

The Ac herein defined refers to a group as follows:

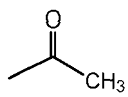

The (E) -MC herein defined refers to a group as follows:

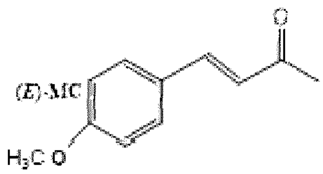

The (Z) -MC herein defined refers to a group as follows:

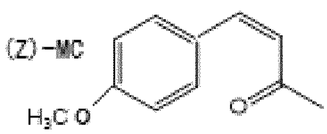

According to a further embodiment, the invention concerns as follows:
According to a further aspect, the "pharmaceutically acceptable salt" hereinafter in the present invention means the compounds are formed by the structural formula I and alkalis or any metal ions including sodium, potassium, calcium, aluminum, copper, zinc and magnesium; The said "alkali" includes sodium hydroxide, potassium hydroxide, calcium hydroxide, magnesium hydroxide, sodium bicarbonate or ammonia; The said "alkaline earth metals" include sodium,

potassium, calcium, aluminum, copper, zinc or magnesium.

According to a further aspect, the said "compound" refers to the structural formula as follows:

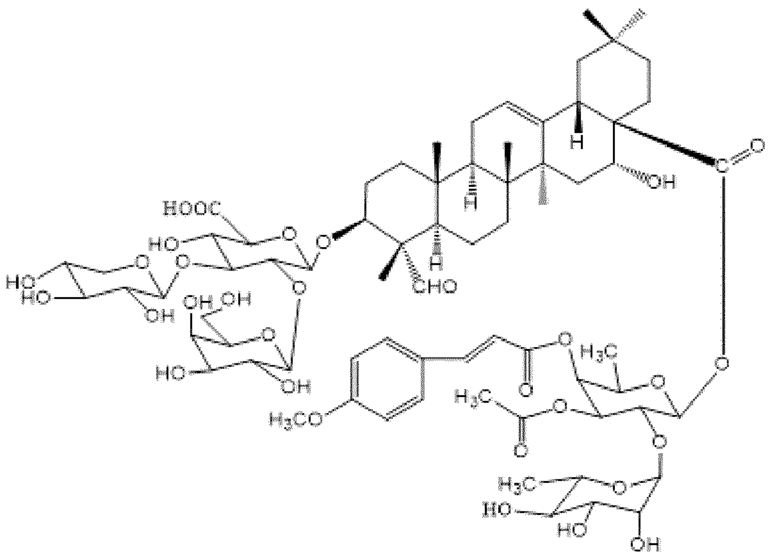

Compound (1)

According to a further aspect, the said "compound" refers to the structural formula as follows:

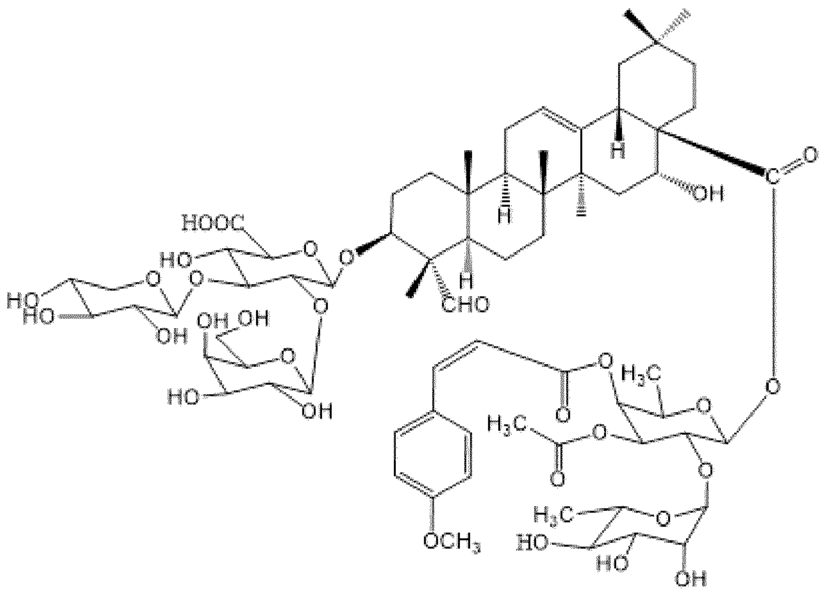

Compound (2)

According to a further aspect, the said "compound" refers to the structural formula as follows:

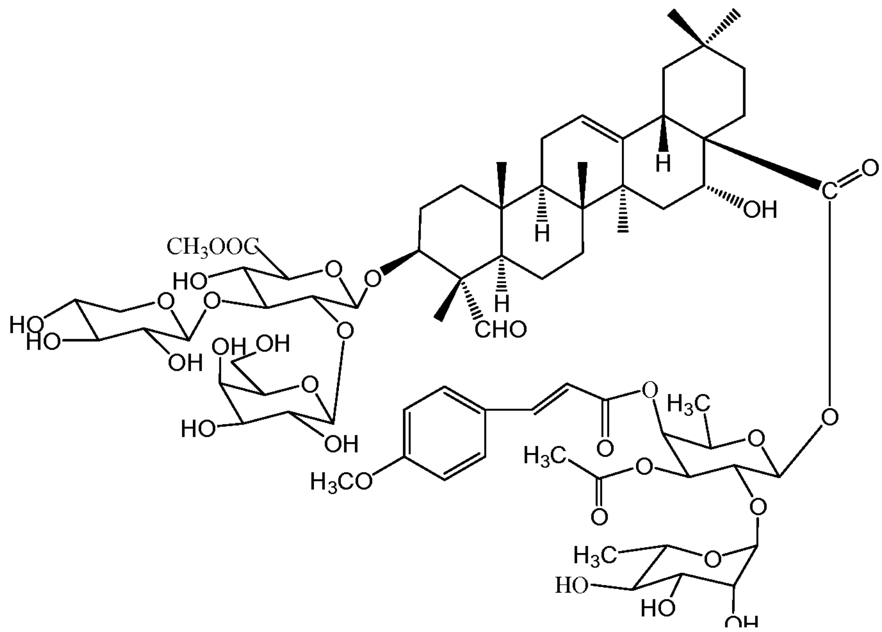

Compound (3)

According to a further aspect, the said "compound" refers to the structural formula as follows:

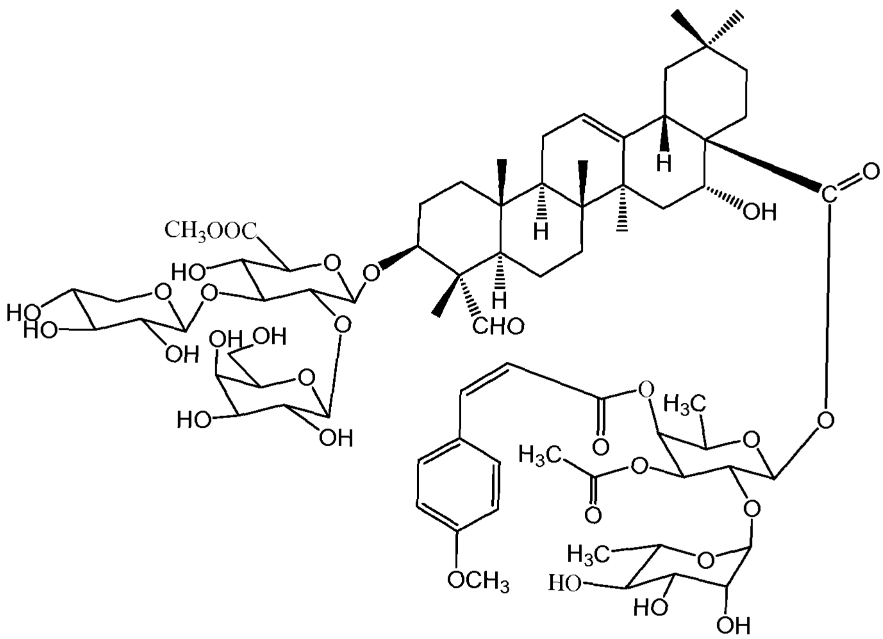

Compound (4)

According to a further aspect, a reference compound which does not form part of the present invention has the structural formula as follows:

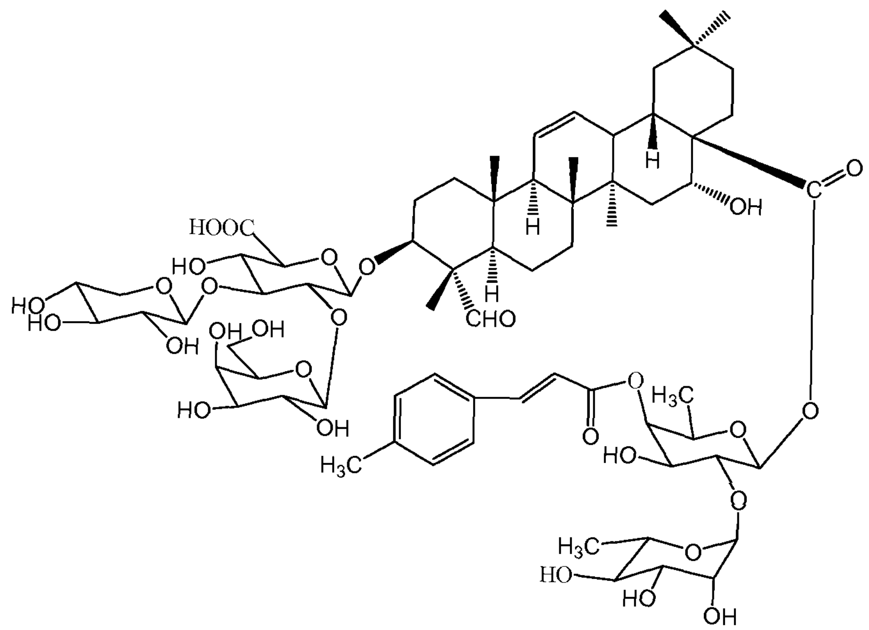

Compound (5)

According to a further aspect, another reference compound which does not form part of the present invention has the structural formula as follows:

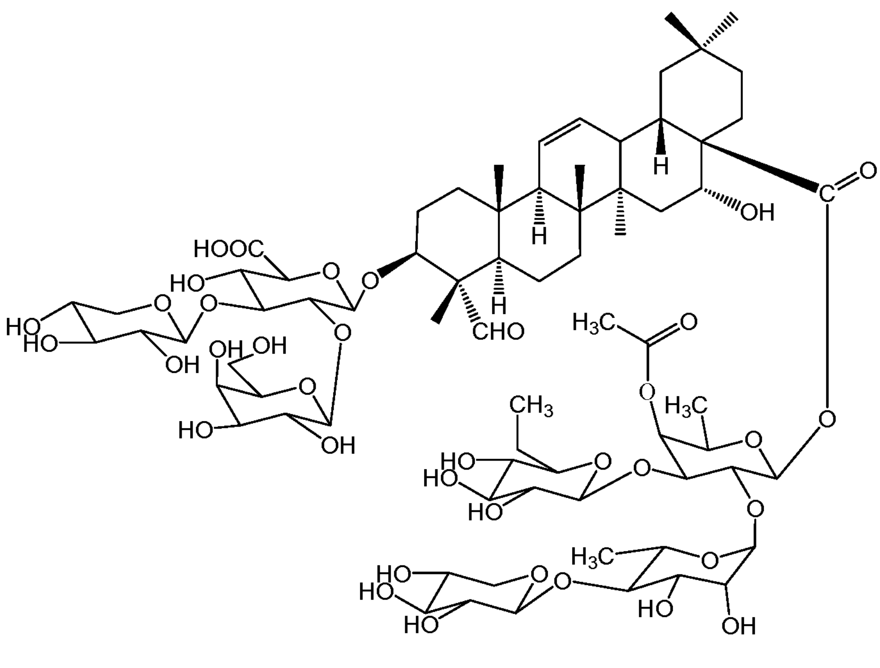

Compound (6)

According to a further aspect, the said "compound" refers to the structural formula as follows:

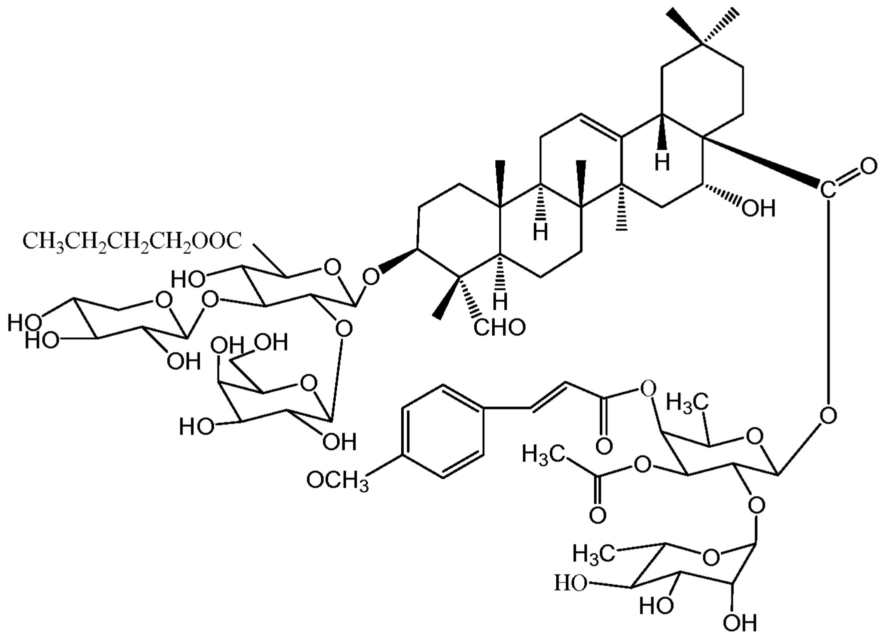

Compound (7)

According to a further aspect, the said "compound" refers to the structural formula as follows:

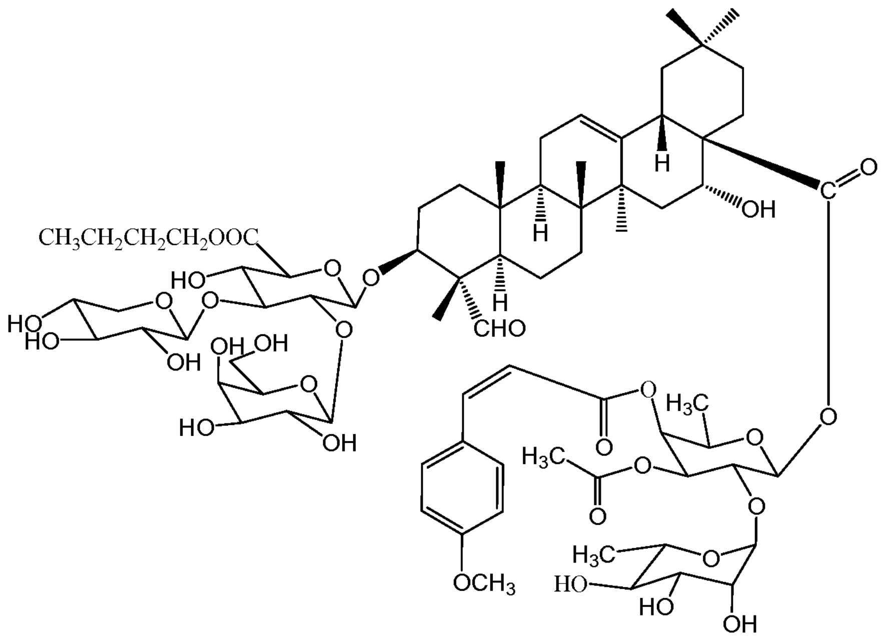

Compound (8)

According to a further aspect, the invention relates to the formula I and compound (1), (2), (3), (4), (7) and (8) for use in the treatment of liver cancer, gastric cancer, colon cancer, breast cancer or melanoma.

According to a further aspect, the dosage range of the said formula I and Compound (1), (2), (3), (4), (7) and (8) is 0.1 to 10 mg/kg animal body weight. It can be converted into clinical dosage for patients according to the common sense.

According to a further aspect, the said formula I and Compound (1), (2), (3), (4), (7) and (8) may be used alone, a mixture of two or more, or mixed with other auxiliary materials into preparations which herein defined include injections, tablets, capsules, granules, dropping pills, food or beverage.

According to a further aspect, the said herein injections include intravenous injection, intramuscular injection, hypodermic injection, intradermal injection and cavity injection and other injections.

According to a further aspect, the present invention provides an anti-tumor composition of which the active ingredients are composed of compounds of formula I or salts of compounds of formula I and any one of the conventional anticancer drugs.

According to a still further aspect, the said "conventional anticancer drugs" means the first-line or the second-line anticancer drugs commonly used in clinics, such as 5-fluorouracil, cyclophosphamide, etoposide, doxorubicin, paclitaxel, irinotecan, oxaliplatin, cisplatin or gemcitabine.

According to a still further aspect, the present invention provides an application of the composition in preparation of the medicament for the treatment of tumors. The composition is composed of compounds of formula I and other anti-tumor drugs or adjuvant anti-tumor drugs.

The present invention said "compound" of structural formula I were mainly extracted from *Silene viscidula,* and the same structural compounds and their analogues extracted from other plants, or obtained by chemical synthesis, semi synthetic or biological transformation methods are also within the scope of the invention.

The present study of the invention showed that the compounds of pentacyclic trieterpenoid saponins from *silene viscidula* have strong inhibitory effects on proliferation of the human hepatoma cell line, human gastric carcinoma cell line, human colon cancer cell line, human breast cancer cell line and melanoma cell line. Among them, the compounds are most sensitive to human hepatoma cell line. At the same concentration level, the compounds' inhibitory effect is better than that of the positive control drug of Cisplatin and cyclophosphamide.

Moreover, the study of the present invention showed that the compounds can improve living state and weight growth of the tumor-bearing nude mice which indicated that the compounds have less side effects compared with other chemotherapeutic agents.

## Experimental Examples

Hereinafter, the principles and features of this invention will be illustrated by reference to examples which are only for explaining the present invention, but are not intended to limit the protection scope of the invention.

### Example 1

### Preparation of *Wacao* total saponins

The roots of *Silene viscidula* were dried and grinded into powder. 21kg powder was extracted with 95 % and 70% ethanol three times under reflux and acquired 7 kg extracts. The extracts were diluted with water, then added to the D101 macroporous adsorption resin and eluted with 100% distilled water, 10% ethanol, 70% ethanol and 90% ethanol respectively. The 90% ethanol elution part was the said total saponins. The content of total saponins was determined by spectrophotometry and the content was 61.2%.

### Example 2

### Preparation of pentacyclic trieterpenoid saponins of *Silene viscidula*

21kg powder of *Silene viscidula* roots was extracted with 170L of 95 % and 70% ethanol three times under reflux and acquired 7 kg extracts. The extracts were diluted with water, then added to the D101 macroporous adsorption resin and eluted with 100% distilled water, 10% ethanol, 70% ethanol, and 90% ethanol respectively. The 90% ethanol elution fraction was dried and obtained the total saponins 5kg. The total saponins were separated by Sephadex LH-20 column and MCI column with 100 % distilled water, 50%, 60%, 70%, 90%, 95% and 100% methanol elution. The elutions were repeatedly subjected to Sephadex LH-20 column chromatography and six compounds were isolated and identified as sinocrassuloside VI, sinocrassuloside VII, sinocrassuloside VIII, sinocrassuloside IX, sinocrassuloside XII, and sinocrassuloside X III. Among them, sinocrassuloside VI and sinocrassuloside VII, sinocrassuloside VIII and sinocrassuloside IX, sinocrassuloside XII and sinocrassuloside XIII are the three pairs of cis-trans isomers. Chemical structure identification data are as follows:

Compound (1) or (2) were obtained as white amorphous powders, ESI-MS (m/z): 1473.2 [M + Na]+, 1449.7[M-H]$^-$ ; $^1$H-NMR and $^{13}$C-NMR: Table1. The ESI-MS (m/z) of Compound (1) or (2) showed a molecular ion at m/z 1450 corresponding to the same molecular formula $C_{71}H_{102}O_{31}$. It was unambiguously identified as sinocrassuloside VI and sinocrassuloside VII on the basis of its $^1$H-NMR and $^{13}$C -NMR spectral data (Table 1).

Compound (3) and (4) were obtained as white amorphous powders, ESI-MS (m/z): 1487.2 [M + Na]$^+$, 1499.7 [M +

Cl]-, 1463.8 [M-H]-. $^1$H-NMR and $^{13}$C-NMR: Table1. The ESI-MS (m/z) of Compound (3) and (4) showed a molecularion at m/z 1464 corresponding to the same molecular formula $C_{72}H_{104}O_{31}$. It was unambiguously identified as sinocrassuloside VIII and sinocrassuloside IV on the basis of their $^1$H-NMR and $^{13}$C -NMR spectral data (Table1). Compound (7) and (8) were obtained as white amorphous powders, ESI-MS(m/z): 1529.3[M+Na]+, 1541.8[M+Cl]-, 1505.6[M-H]-; $^1$H-NMR and $^{13}$C-NMR: Table1. The ESI-MS (m/z) of Compound (7) and (8) showed the same molecular formula $C_{75}H_{110}O_{31}$. It was unambiguously identified as sinocrassulosideXII and sinocrassulosideXIII on the basis of their $^1$H-NMR and $^{13}$C -NMR spectral data (Table 1).

Table 1 $^1$H-NMR and $^{13}$C-NMR data of Compound (1), (2), (3), (4), (7) and (8)

| | Compound (1) δHmult (J in Hz)[a]) δC[b]) | | Compound (2) δHmult (J in Hz)[a]) δC[b]) | | Compound (3) δHmult (J in Hz)[a]) δC[b]) | | Compound (4) δHmult (J in Hz)[a])δC[b]) | | Compound (7) δHmult (J in Hz)[a]) δC[b]) | | Compound (8) δHmult (J in Hz)[a]) δC[b]) | |
|---|---|---|---|---|---|---|---|---|---|---|---|---|
| The aglycone moiety | | | | | | | | | | | | |
| 1 | 0.81,1.37 | 37.8 | 0.82,1.37 | 37.8 | 0.85,1.39 | 37.8 | 0.85,1.39 | 37.8 | 0.86,1.40 | 37.7 | 0.86,1.40 | 37.7 |
| 2 | 1.82,2.09 | 24.8 | 1.82,2.09 | 24.8 | 1.81,2.01 | 24.7 | 1.81,2.01 | 24.7 | 1.83,2.02 | 24.9 | 1.82,2.029 | 24.9 |
| 3 | 3.94(7.8) | 84.2 | 3.94 | 84.2 | 4.09 | 84 | 4.09 | 84 | 4.1 | 84 | 4.1 | 84.1 |
| 4 | | 54.3 | | 54.3 | | 54.3 | | 55.3 | | 54.2 | | 54.2 |
| 5 | 1.35 | 48.5 | 1.35 | 48.5 | 1.36 | 48.5 | 1.36 | 48.5 | 1.37 | 48.4 | 1.37 | 48.4 |
| 6 | 0.88,1.37 | 20.2 | 0.88,1.37 | 20.2 | 0.89,1.36 | 20.2 | 0.89,1.36 | 20.2 | 0.89,1.38 | 20.3 | 0.89,1.38 | 20.2 |
| 7 | 1.5 | 32.2 | 1.5 | 32.2 | 1.51 | 32.1 | 1.51 | 32.1 | 1.52 | 32.1 | 1.52 | 32 |
| 8 | | 40.5 | | 40.3 | | 40.4 | | 40.4 | | 40.4 | | 40.3 |
| 9 | 1.78 | 46.3 | 1.78 | 46.3 | 1.78 | 46.8 | 1.76 | 46.8 | 1.78 | 46.7 | 1.77 | 46.7 |
| 10 | | 35.3 | | 35.3 | | 35.8 | | 35.9 | | 35.9 | | 35.8 |
| 11 | 1.91 | 23.3 | 1.91 | 23.3 | 1.91 | 23.3 | 1.91 | 23.3 | 1.92 | 23.2 | 1.92 | 23.2 |
| 12 | 5.57 brs | 121.8 | 5.57 br s | 121.8 | 5.60 br s | 121.8 | 5.60 br s | 121.8 | 5.61 brs | 122 | 5.61 br s | 122 |
| 13 | | 143.6 | | 143.5 | | 143.6 | | 143.6 | | 143.7 | | 143.6 |
| 14 | | 41.5 | | 41.5 | | 41.5 | | 41.5 | | 41.6 | | 41.7 |
| 15 | 1.90,2.18 | 35.8 | 1.90,2.16 | 35.9 | 1.95,2.18 | 35.8 | 1.91,2.18 | 35.9 | 1.96,2.20 | 35.9 | 1.96,2.20 | 36 |
| 16 | 5.20 brs | 73 | 5.17br s | 73 | 5.22 br s | 73.1 | 5.20 br s | 73.1 | 5.23 brs | 73.1 | 5.19br s | 73.2 |
| 17 | | 49 | | 49 | | 48.5 | | 48.5 | | 48.5 | | 48.5 |
| 18 | 3.39 d (13.8) | 40.9 | 3.38 | 40.9 | 3.39 d (14.0) | 40.9 | 3.39 d (14.0) | 40.9 | 3.40 d (14.0) | 40.9 | 3.4 | 40.9 |
| 19 | 1.34,2.74 t (14.0) | 47.9 | 1.34,2.74 t | 47.9 | 1.34,2.75 t (14.4) | 47.9 | 1.36,2.75 t (14.4) | 47.9 | 1.36,2.76 t (14.4) | 48 | 1.36,2.76 t (14.4) | 48 |
| 20 | | 30.6 | | 30.5 | | 30.5 | | 30.6 | | 30.5 | | 30.5 |
| 21 | 1.30,2.40 | 35.2 | 1.32,2.41 | 35.2 | 1.30,2.41 | 35.2 | 1.32,2.41 | 35.3 | 1.30,2.40 | 35.3 | 1.32,2.41 | 35.3 |
| 22 | 2.22,2.39 | 32.2 | 2.20,2.38 | 32.2 | 2.22,2.40 | 32.1 | 2.20,2.40 | 32.1 | 2.23,2.40 | 32 | 2.23,2.40 | 32 |
| 23 | 9.84 s | 210.5 | 9.84s | 210.5 | 9.86s | 210.5 | 9.86s | 210.5 | 9.88 s | 210.6 | 9.88s | 210.6 |
| 24 | 1.39s | 10.7 | 1.39s | 10.7 | 1.39 s | 10.7 | 1.40s | 10.7 | 1.41s | 10.7 | 1.41s | 10.7 |
| 25 | 0.79 s | 15.8 | 0.84 s | 15.8 | 0.84 s | 15.8 | 0.88 s | 15.8 | 0.88 s | 15.7 | 0.88 s | 15.8 |
| 26 | 1.03s | 17.1 | 1.04s | 17.1 | 1.07 s | 17.1 | 1.07 s | 17.2 | 1.08s | 17 | 1.08 s | 17 |

(continued)

| | Compound (1) δHmult (J in Hz)a) δCb) | | Compound (2) δHmult (J in Hz)a) δCb) | | Compound (3) δHmult (J in Hz)a) δCb) | | Compound (4) δHmult (J in Hz)a)δCb) | | Compound (7) δHmult (J in Hz)a)δCb) | | Compound (8) δHmult (J in Hz)a) δCb) | |
|---|---|---|---|---|---|---|---|---|---|---|---|---|
| 27 | 1.75 s | 26.7 | 1.77 s | 26.7 | 1.79 s | 26.7 | 1.76 s | 26.7 | 1.79 s | 26.8 | 1.79 s | 26.8 |
| 28 | | 175.1 | | 175.1 | | 175.1 | | 175.1 | | 175 | | 175.1 |
| 29 | 0.94s | 33.2 | 0.94s | 33.2 | 0.96 s | 33.2 | 0.98 s | 33.2 | 0.98 s | 33.3 | 0.99 s | 33.2 |
| 30 | 0.98 s | 24.7 | 0.99 s | 24.6 | 1.02 s | 24.6 | 1.02 s | 24.6 | 1.03s | 24.5 | 1.02 s | 24.4 |
| 3-*0*-*B*-D-Glucuronopyranosyl | | | | | | | | | | | | |
| 1' | 4.88 d (7.2) | 102.6 | 4.88 d (7.2) | 102.6 | 4.86 d (7.8) | 102.6 | 4.86 d (7.8) | 102.6 | 4.87 d (7.2) | 102.6 | 4.88 d (7.2) | 102.6 |
| 2' | 4.34 t (9.0) | 77.4 | 4.34 | 77.4 | 4.36 | 77.4 | 4.36 | 77.4 | 4.36 t (9.0) | 77.3 | 4.36 | 77.3 |
| 3' | 4.28 t (9.0) | 84.2 | 4.27 | 84.2 | 4.29 | 84.2 | 4.27 | 84.2 | 4.30 t (9.0) | 84.1 | 4.3 | 84.2 |
| 4' | 4.44 | 70.2 | 4.44 | 70.2 | 4.24 | 69.9 | 4.24 | 69.8 | 4.25 | 70.1 | 4.25 | 70 |
| 5' | 4.5 | 77 | 4.5 | 77 | 4.39 | 75.3 | 4.39 | 75.3 | 4.42 | 75.2 | 4.42 | 75.1 |
| 6' | | 170.4 | | 170.4 | | 169.5 | | 169.5 | | 179.8 | | 179.8 |
| 7' | | | | | 3.72 s | 52.4 | 3.72 s | 52.4 | 4.31 t (6.6) | 66.5 | 4.33 t (6.6) | 66.5 |
| 8' | | | | | | | | | 1.64 m | 28.3 | 1.67 m | 28.4 |
| 9' | | | | | | | | | 1.25 m | 22.5 | 1.28 m | 22.6 |
| 10' | | | | | | | | | 0.85 t (7.2) | 14.3 | 0.86 t (7.2) | 14.2 |
| 2'-O-β-D-Galactopyranosyl | | | | | | | | | | | | |
| 1" | 5.55 d (4.2) | 102.7 | 5.55 d (4.2) | 102.7 | 5.54 d (7.8) | 102.7 | 5.54 d (7.8) | 102.7 | 5.56 d (7.8) | 102.6 | 5.55 d (7.8) | 102.6 |
| 2" | 4.46 | 72 | 4.46 | 72 | 4.47 | 73 | 4.46 | 73 | 4.47 | 73.1 | 4.47 | 73.1 |
| 3" | 4.14 dd (7.8,3.6) | 6) 74 | 4.14 dd (7.8,3.6) | 74 | 4.14 dd | 74.9 | 4.14 dd | 74.9 | 4.15 dd (7.2,3.0) | 74.8 | 4.14 dd (7.2,3.0) | 74.9 |
| 4" | 4.55 | 68.9 | 4.55 | 68.9 | 4.57 | 68.5 | 4.57 | 68.5 | 4.56 | 68.4 | 4.56 | 68.4 |
| 5" | 4.02 | 75.3 | 4.02 | 75.3 | 4.02 | 77 | 4.02 | 77 | 4.02 | 77.1 | 4.02 | 77.1 |
| 6" | 4.43,4.50 | 60.2 | 4.42,4.50 | 60.2 | 4.44,4.52 | 60.3 | 4.44,4.51 | 60.3 | 4.45,4.51 | 60.2 | 4.45,4.51 | 60.2 |
| 3'-O-β-D-Xylopyranosyl | | | | | | | | | | | | |
| 1''' | 5.31d (7.8) | 103.5 | 5.31 d (7.8) | 103.5 | 5.30 d (7.8) | 103.6 | 5.30 d (7.8) | 103.6 | 5.32d (7.8) | 103.6 | 5.32 d (7.8) | 103.6 |
| 2''' | 3.94 t (7.8) | 73.8 | 3.94 t (7.8) | 73.8 | 3.94 t (7.8) | 74.1 | 3.93 t (8.4) | 75.3 | 3.95 t (7.8) | 74.2 | 3.94 5 (7.8) | 74.2 |
| 3''' | 4.08 | 77.4 | 4.08 | 77.4 | 4.08 | 77.4 | 4.07 | 77.4 | 4.09 | 77.4 | 4.09 | 77.4 |
| 4''' | 4.1 | 70 | 4.1 | 70 | 4.1 | 68.9 | 4.09 | 68.9 | 4.1 | 69.1 | 4.1 | 69.1 |
| 5''' | 3.57,4.21 | 66.2 | 3.57,4.20 | 66.2 | 3.64,4.21 | 66.2 | 3.62,4.21 | 66.2 | 3.66,4.22 | 66.3 | 3.66,4.22 | 66.2 |

(continued)

| | Compound (1) δHmult (J in Hz)[a]) δC[b]) | | Compound (2) δHmult (J in Hz)[a]) δC[b]) | | Compound (3) δHmult (J in Hz)[a]) δC[b]) | | Compound (4) δHmult (J in Hz)[a])δC[b]) | | Compound (7) δHmult (J in Hz)[a]) δC[b]) | | Compound (8) δHmult (J in Hz)[a]) δC[b]) | |
|---|---|---|---|---|---|---|---|---|---|---|---|---|
| 28-O-β-D-Fucopyranosyl | | | | | | | | | | | | |
| 1”” | 6.17 d (8.4) | 93.2 | 6.13d (8.4) | 93.1 | 6.19d (7.8) | 93.2 | 6.14 d (7.8) | 93.2 | 6.20 d (8.4) | 93.3 | 6.15d (8.4) | 93.2 |
| 2”” | 4.71 t (8.4) | 71.3 | 4.62 t (8.4) | 71.3 | 4.72t (9.6) | 70.5 | 4.62 t (9.0) | 70.6 | 4.73 t (9.0) | 70.5 | 4.70 t (9.0) | 70.5 |
| 3”” | 5.68 dd (9.3,4.8) | 73.1 | 5.68 dd (9.3,4.8) | 73.1 | 5.70 dd | 73.8 | 5.69dd | 73.8 | 5.72 dd | 73.9 | 5.72dd | 74 |
| 4”” | 5.75 | 70.1 | 5.75 | 70.1 | 5.75 | 69.9 | 5.77 | 69.8 | 5.76 | 69.8 | 5.76 | 69.8 |
| 5”” | 4.2 | 69.9 | 4.21 | 68.5 | 4.19 | 68.5 | 4.2 | 68.5 | 4.21 | 68.5 | 4.21 | 68.5 |
| 6”” | 1.22 d (6.6) | 16.1 | 1.19 d (6.6) | 16.1 | 1.24 d (6.0) | 16.1 | 1.21 d (6.6) | 16.1 | 1.26 d (6.6) | 16.3 | 1.25 d (6.6) | 16.3 |
| 2””-O-α-L-Rhamnopyranosyl | | | | | | | | | | | | |
| 1””’ | 5.76 s | 101.6 | 5.75s | 101.6 | 5.77 s | 101.7 | 5.75s | 101.7 | 5.78 s | 101.7 | 5.76s | 101.8 |
| 2””’ | 4.52 | 70.5 | 4.52 | 70.5 | 4.54 | 70.2 | 4.53 | 70.2 | 4.54 | 70.4 | 4.54 | 70.3 |
| 3””’ | 4.36 | 70.6 | 4.36 | 68.5 | 4.36 | 71.9 | 4.35 | 71.9 | 4.37 | 72 | 4.36 | 72 |
| 4””’ | 4.23 | 71.9 | 4.23 | 71.9 | 4.24 | 72 | 4.24 | 72 | 4.24 | 72.2 | 4.24 | 72.2 |
| 5””’ | 4.4 | 70 | 4.4 | 70 | 4.44 | 68.9 | 4.44 | 68.9 | 4.46 | 68.7 | 4.45 | 68.6 |
| 6””’ | 1.62 d (6.0) | 18.4 | 1.62 d (6.0) | 18.4 | 1.65 d (6.6) | 18.4 | 1.65d (6.6) | 18.4 | 1.66 d (6.6) | 18.8 | 1.65 d (6.6) | 18.8 |
| The acetyl group | | | | | | | | | | | | |
| 1””” | | 169.9 | | 169.8 | | 169.9 | | 169.9 | | 170.2 | | 170.1 |
| 2””” | 2.00 s | 20.9 | 1.97s | 20.9 | 2.01 s | 20.9 | 2.00 s | 20.7 | 2.02 s | 21 | 2.00s | 21 |
| The para-methoxycinnamoyl group (MC) | | | | | | | | | | | | |
| 1”””” | | 166.8 | | 166 | | 166.8 | | 166.8 | | 166.8 | | 166.7 |
| 2”””” | 6.60 d (15.6) | 114.7 | 5.91 d (12.9) | 114.8 | 6.61d (16.2) | 114.7 | 5.93 d (13.2) | 114.8 | 6.62 d (15.6) | 114.9 | 5.96d (12.0) | 114.8 |
| 3”””” | 7.95 d (15.6) | 145.9 | 6.95 d (12.6) | 145.9 | 7.96 d (16.2) | 145.9 | 6.96 d (13.2) | 145.9 | 7.97 d (15.6) | 146 | 6.98 d (12.0) | 146 |
| 4”””” | | 126.8 | | 127.1 | | 126.9 | | 127.1 | | 127 | | 127.1 |
| 5”””” | | | | | | | | | | | | |
| & | 7.53 d (12.6) | 130.9 | 7.96d (9.0) | 132.7 | 7.54 d (10.2) | 130.9 | 7.99d (8.4) | 132.7 | 7.55 d (10.8) | 131 | 7.98d (9.0) | 132.8 |
| 9”””” | | | | | | | | | | | | |
| 6”””” | | | | | | | | | | | | |

(continued)

| | | | | | | | | | | | | |
|---|---|---|---|---|---|---|---|---|---|---|---|---|
| The para-methoxycinnamoyl group (MC) | | | | | | | | | | | | |
| & 8''''''' | 7.00 d (8.4) | 114 | 6.95d (9.0) | 114 | 7.02 d (9.0) | 114.8 | 6.97 d (9.0) | 114.8 | 7.03 d (9.0) | 114.9 | 6.94d (9.0) | 114.9 |
| 7''''''' | | 161.7 | | 160.8 | | 161.7 | | 160.8 | | 161.8 | | 160.8 |
| *p*-$OCH_3$ | 3.67 s | 55.7 | 3.64 s | 55.7 | 3.68 s | 55.8 | 3.65s | 55.7 | *3.67 s* | 55.9 | 3.68 s | 55.8 |

Results of chemical structure identification were as follows :

Compound (1) or (2) were obtained as white amorphous powders and the same molecular formula was $C_{71}H_{102}O_{31}$. Their chemical structures were identified as follows:

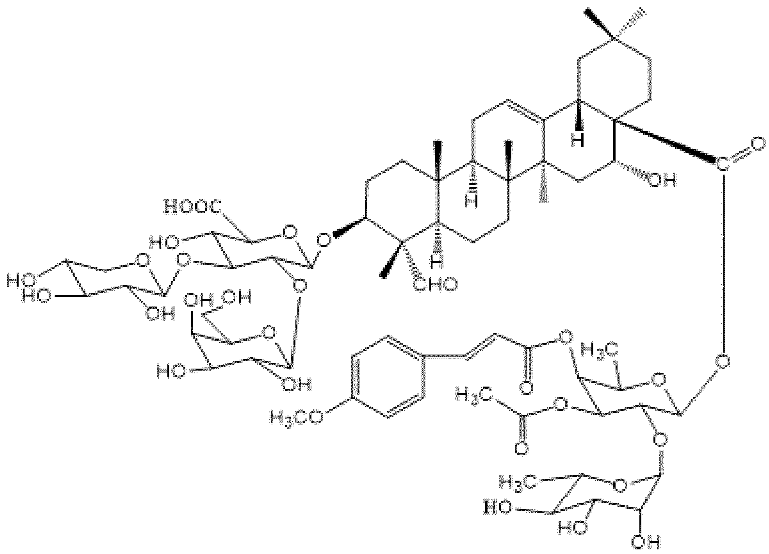

Compound (1)

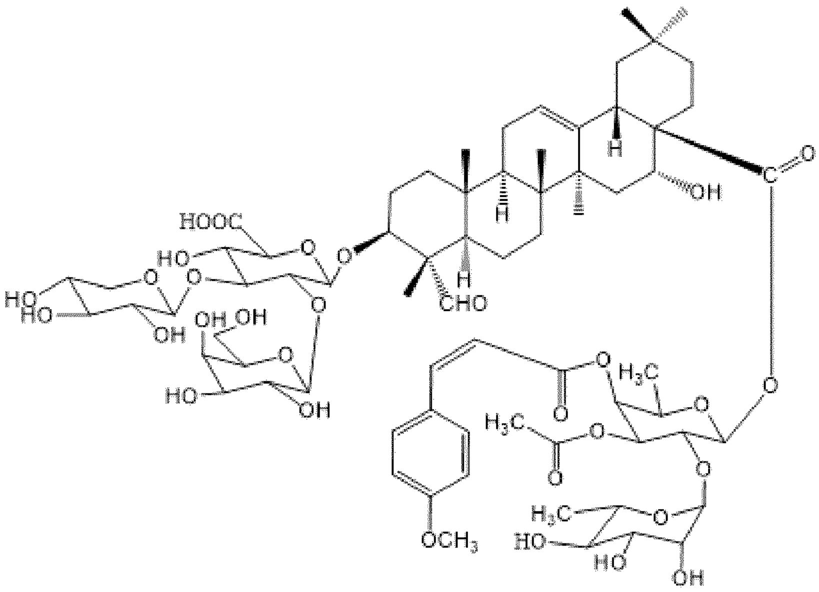

Compound (2)

Compound (3) and (4) were obtained as white amorphous powders and the same molecular formula was $C_{72}H_{104}O_{31}$. Their chemical structures were identified as follows:

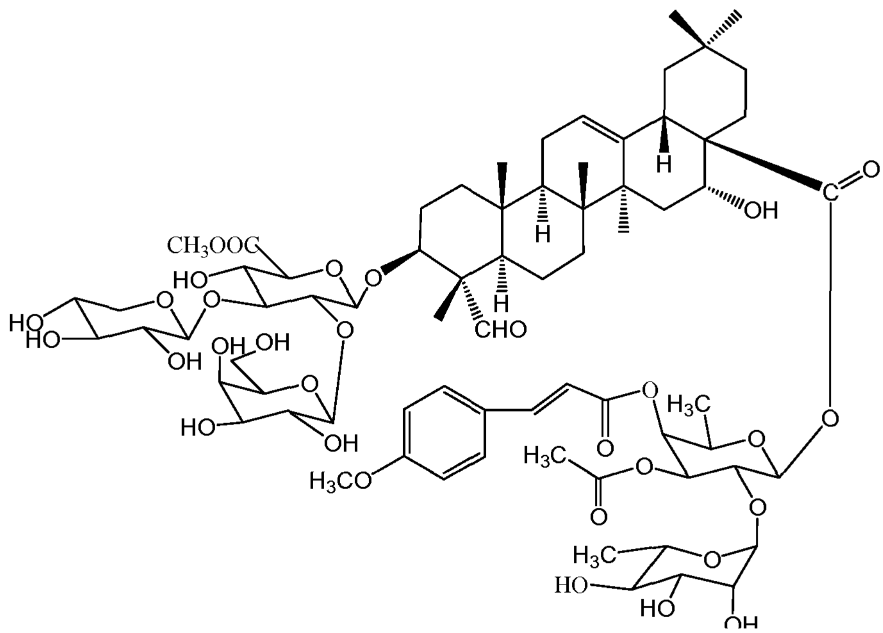

Compound (3)

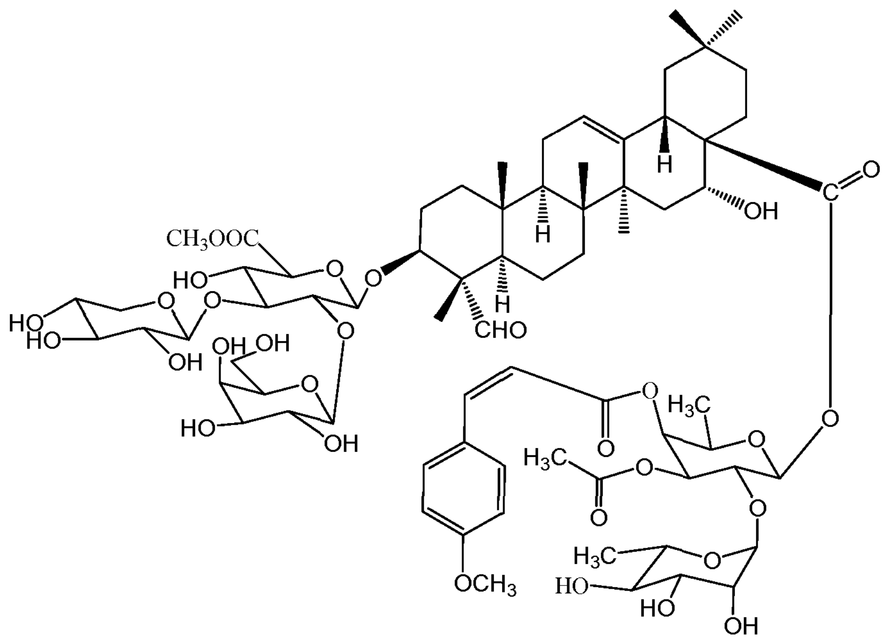

Compound (4)

Compound (7) and (8) were obtained as white amorphous powders and the same molecular formula was $C_{75}H_{110}O_{31}$. Their chemical structures were identified as follows:

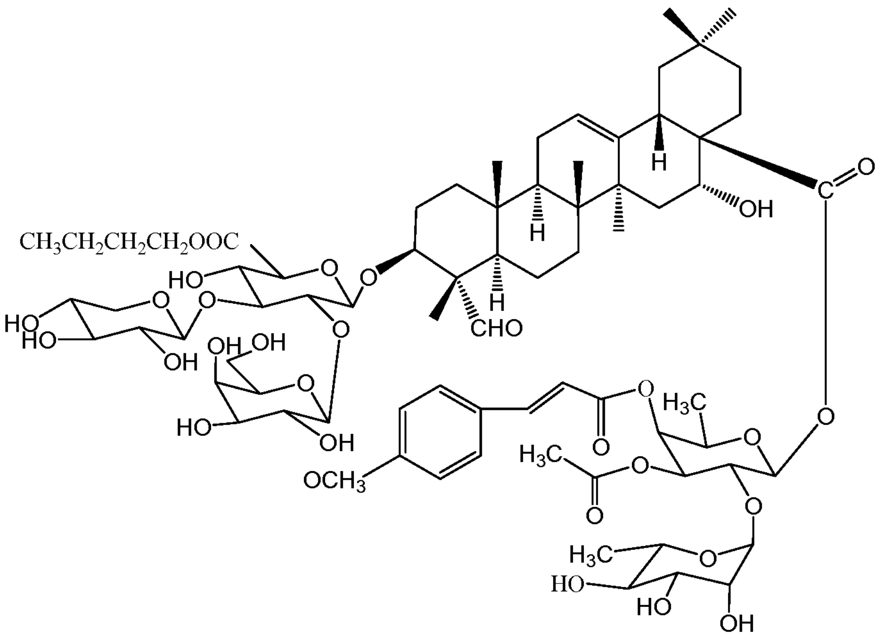

Compound (7)

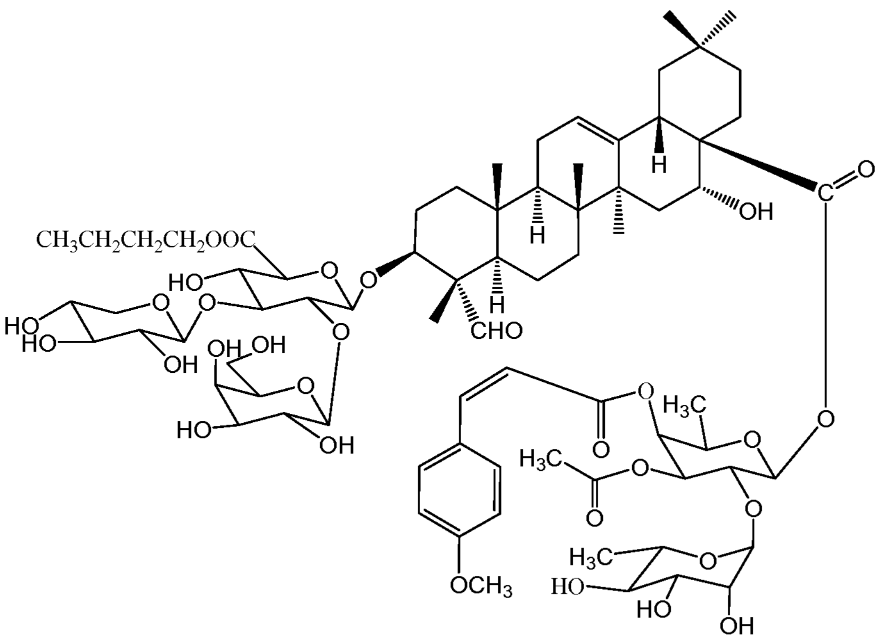

Compound (8)

Example 3

**Effects of *Wacao* saponins on inhibition of different tumor cells in vitro**

**1. Methods**

**1.1 Tumor cell line and culture**

Human resources tumor cell line BGC823, BGC823, HT29, MCF-7 and A875 were provided by Cancer Hospital, Chinese Academy of Medical Sciences. Cells were cultured in RPMI-1640 medium containing 100U/mL Penicillin and

100μg/mL Streptomycin with 10 % fetal bovine serum and maintained at 37 °C in a humidified atmosphere (5 % $CO_2$ and 95% $O_2$).

Cells were passaged every 2 or 3 days, and all the experiments were carried out on exponentially growing cells.

### 1.2 Experimental design

All cells were cultured in a humidified atmosphere (5 % $CO_2$ and 95% $O_2$), and passaged every 2 or 3 days. Cells were harvested on exponentially growing phase, and cell suspension was prepared with $2\times10^4$ cells /mL, added with 100 μL per well of 96-well plates, and cultured for 24 h in incubator. 100 μL medium without cells was added per well as blank group.

Compound (1), (2), (3), (4), (7) or (8) was dissolved in DMSO respectively, and diluted with 1640 medium at a final concentration of 400 μg/mL, so as to that of the Cisplatin. Cells were divided into 9 groups, they were blank group, model group, Compound (1), (2), (3), (4), (7) and (8) groups, and Cisplatin group as the positive one, 6 wells per group.

Before drug administration, supernatant of each well was removed and fresh 1640 medium was added, 180 μL per well. Compounds and Cisplatin were added with 20 μL per well respectively at a final concentration of 40 μg/mL. Wells of blank and model groups were added with 0.5 % DMSO, 20 μL per well. 48 hours later, 20 μL MTT was added per well and continued to culture for 4 hours, then the supernatant was replaced carefully and DMSO was added with 150 μL per well to end the experiment. The 96-well plates were shaken for 10 mins and read the absorbance at 490 nm. The Inhibition rate was calculated.

### 1.3 Inhibition rate calculation

$$\text{Inhibition rate} = [(OD_{model} - OD_{blank}) - (OD_{drugs} - OD_{blank})] / (OD_{model} - OD_{blank}) \times 100\%$$

The MTT experiment was repeated three times.

### 2 Statistical Analysis

Results were expressed as means ± SD. Data analyses were performed using analysis of variance with the SPSS 10.0 software for Windows.

### 3 Results

All types of *Wacao* saponins showed obvious inhibitory activity to HepG2, MCF-7, BGC823, HT29 and A875 cells at a final concentration of 40 μg/mL. After co-cultured with *Wacao* saponins, cells were shrunk, broken or floated obviously. The inhibition rates of Compound (1) or (2) to HepG2, BGC823, and HT29 cells exceeded 90 %, and the inhibitory activity to BGC823 showed the best, the inhibition rate was even up to 99 %. Compound (3), (4), and (5) showed stronger inhibitory effects on tumor cell lines above, by contrast, Compound (7) and (8) showed relatively weaker inhibitory effects, as shown in Table 1.

Table 1 Effects of *Wacao* saponins on inhibition of different tumor cells in vitro

| (n=3 , $\bar{x}\pm s$) | | | | | |
|---|---|---|---|---|---|
| Groups | 抑制率 (%) | | | | |
| | HepG2 | BGC823 | HT29 | MCF-7 | A875 |
| Compound (1) | 99.34±1.01 | 96.82±1.81 | 96.06±5.60 | 83.12±2.33 | 88.02±12.57 |
| Compound (2) | 98.72±1.02 | 96.53±3.72 | 99.02±1.06 | 91.00±8.68 | 77.49±9.94 |
| Compound (3) | 74.35±1.51 | 92.09±1.13 | 56.62±4.24 | 90.12±7.43 | 68.35±5.37 |
| Compound (4) | 65.68±1.05 | 92.43±6.24 | 90.62±5.60 | 56.25±1.96 | 70.51±9.76 |
| Compound (7) | 21.83±1.53 | 77.08±5.73 | 62.67±3.12 | 58.12±6.26 | 55.64±9.88 |
| Compound (8) | 41.25±1.27 | 56.00±7.90 | 77.22±6.17 | 61.32±11.67 | 55.32±13.35 |

(continued)

| (n=3 , $\bar{x}\pm s$) | | | | | |
|---|---|---|---|---|---|
| Groups | 抑制率 (%) | | | | |
| | HepG2 | BGC823 | HT29 | MCF-7 | A875 |
| Cisplatin | 82.15±4.05 | 97.86±4.12 | 99.04±1.15 | 97.53±2.05 | 90.13±1.98 |
| Note: n=3 means that the MTT experiment was repeated three times. | | | | | |

**Discussion**

In this experiment, we compared the anti-tumor activity of different compounds with each other. It was indicated that Compound (1) or (2) (cis-trans-isomers) had stronger anti-tumor activity, and followed by Compound (3) and (4) (cis-trans-isomers), and Compound (7). Among them, the activity of Compound (2) showed the best. Hepatoma is the most sensitive one in these 5 types of cell lines above, and the inhibitory activity to hepatoma is better than that of the Ciplatin. This result provided much more experimental basis and references for the following animal experiments.

**Example 4**

**The inhibitory activity and $IC_{50}$ of Component (2) to HepG2 at different time of treatment**

**1. Methods**

1.1 HepG2 cell line culture

Human resources hepatoma cell line HepG2 was provided by Cancer Hospital, Chinese Academy of Medical Sciences. Cells were cultured in RPMI-1640 medium containing 100U/mL Penicillin and 100μg/mL Streptomycin with 10 % fetal bovine serum at 37 °C and in a humidified atmosphere(5 % $CO_2$ and 95% $O_2$). Cells were passaged every 2 or 3 days, and all the experiments were carried out on exponentially growing cells.

1.2 Experimental design

Cells above were maintained at 37 °C in a humidified atmosphere (5 % $CO_2$ and 95% $O_2$), and passaged every 2 or 3 days. Cells were harvested in good growth status and cell suspension was prepared with $2\times10^4$ cells /mL, added 100 μL per well of 96-well plates, and cultured for 24 h in the incubator. 100 μL liquid medium with no cell was added per well as blank group.

Compound (2) (prepared by the method mentioned in Embodiment 2) was dissolved in DMSO, and diluted with 1640 medium into different concentrations. Cells were divided into blank group, model group, Compound (2) group, and Cisplatin group (positive contrast group), 6 wells per group. 3 plates were used as parallel test to culture cells at different period.

Before medication, supernatant of each well was removed and fresh 1640 medium was added, 180 μL per well. Compound (2) group cells were added with 20 μL different concentrations of Compound (2) solution per well at a final concentrations of 80 μg/mL, 40 μg/mL, 20 μg/mL, 10 μg/mL and 5 μg/mL respectively, and the final concentration of Cisplatin was 40 μg/mL. Wells of blank and model groups were added with 0.5 % DMSO, 20 μL per well. After finished adding drugs, cells were incubated for 24 and 48 h respectively.

At the end of incubation, 20 μL MTT was added per well to end the experiment. The supernatant was removed carefully and 150 μL DMSO was added per well. After shaken for 10 mins, the absorbance was read at 490 nm and the inhibition rate was calculated with the formula below.

1.3 Inhibition rate calculation

$$\text{Inhibition rate} = [\,(OD_{model} - OD_{blank}) - (OD_{drugs} - OD_{blank})\,] / (OD_{model} - OD_{blank}) \times 100\%$$

$IC_{50}$ was calculated with Bliss mothod.
The MTT experiment was repeated three times.

**2 Statistical Analysis**

Results were expressed as means ± SD. Data analyses were performed using analysis of variance with the SPSS 10.0 software for Windows.

**3 Results**

Data showed that obvious inhibitory activity of Compound (2) to HepG2 at different concentration (20-80 μg/mL), and the highest inhibition rate is above 99%. Cells showed obvious shrunken, broken or floating abnormal phenomena. With the concentration of 10 μg/mL, the inhibition rates of Compound (2) to HepG2 cells at 24 and 48 h incubating time-point exceeded 80 %.
At the same time, the $IC_{50}$ of Compound (2) to HepG2 was calculated. The $IC_{50}$ of 24 h time point is 5.25 nmol/mL and 48 h time point is 4.56 nmol/mL, which **showed a certain temporal correlation.**

Table 2 The inhibitory activity and $IC_{50}$ of component (2) to HepG2 at different time of drug treatment

| (n=3, $\bar{x}$ ±SD) | | | |
|---|---|---|---|
| Group | Conc. (μg/ml) | Inhibition Rate (%) | |
| | | 24 h | 48 h |
| Compound (2) | 80 | 94.64±2.59 | 98.89±0.68 |
| | 40 | 89.98±2.41 | 98.40±1.00 |
| | 20 | 92.66±2.43 | 99.47±0.56 |
| | 10 | 83.27±5.56 | 88.17±4.81 |
| | 5 | 13.37±5.76 | 16.65±8.00 |
| Cisplatin | 40 | 47.94±6.328 | 99.45±0.36 |
| Note: Note: n=3 means that the MTT experiment was repeated three times. | | | |

**Example 5**

**Inducing effects and mechanisms of *Wacao* saponin on apoptosis of human hepatoma cells**

**1 Methods**

1.1 HepG2 cell line culture

Human resources hepatoma cell line HepG2 was provided by Cancer Hospital, Chinese Academy of Medical Sciences. Cells were cultured in RPMI-1640 medium containing 100U/mL Penicillin and 100μg/mL Streptomycin with 10 % fetal bovine serum and maintained at 37 °C in a humidified atmosphere(5 % $CO_2$ and 95% $O_2$). Cells were passaged every 2 or 3 days, and all the experiments were carried out on exponentially growing cells.

1.2 Experimental design

1.2.1 Detection of apoptosis

Cells were cultured in vitro for 24 h, then digested, harvested and divided into 3 groups (3 flasks per group), the Model group, Compound (2) group (at a final concentration of 80 μg/mL) and Cisplatin group (at a final concentration of 40 μg/mL) as positive control. Following the incubation of cells in the flasks with drug for 24 hours, cell suspension was harvested with $5\times10^5$ to $1\times10^6$ cells /mL, and taken with 1 mL for centrifuging for 10 min at 1000 RPM and 4 °C. The supernatant was replaced carefully, added with 1 mL cold phosphate-buffered saline, gently shaken for resuspension, recentrifuged and replaced the supernatant. The cells were resuspended with 200 μl Binding Buffer, added with 10 μl Annexin V-FITC and 5 μl PI, gently shaken and reacted for 15 min at Room temperature, then added with 300 μl Binding

Buffer and finally measured immediately with Flow cytometry.

1.2.2 Cell cycle detection

Cells were cultured in flasks and divided into 4 groups, the Model group, Compound (2) groups (at a final concentration of 20, 40 and 80 μg/mL respectively), 3 flasks per group. Following the incubation of cells in the flasks with drug for 24 hours, single cell suspension was prepared and washed two times with phosphate-buffered saline, digested with Trypsin, and ended digestion with medium. Cells were blown and centrifuged at 1000 RPM for 5 mins. The supernatant was removed and 1-2 mL cold phosphate-buffered saline was added, blown and centrifuged again. The precipitate was blew and then fixed with -20 °C 70 % alcohol (1-2 mL), centrifuged again. Alcohol was removed, and the precipitate was washed two times with phosphate-buffered saline, blown for suspension and filtered through 100 mesh gauze filter to flow detection tube, centrifuged at 1000RPM for 5 mins. The supernatant was abandoned and cells were stained with PI dye (50 μg/mL), vibrated and stained at least 30 mins in darkness, detected with Flow cytometry.

## 2 Statistical Analysis

Results were expressed as means ± SD. Data analyses were performed using analysis of variance with the SPSS 10.0 software for Windows.

## 3 Results

3.1 Early apoptosis signal detection

Data showed that early apoptosis signal was significantly enhanced from 1.5 % to 8.5% ($p$<0.01, *vs* Control group), and the number of necrotic cells increased from 2.0% to 6.6%. These results indicated that Compound (2) killed tumor cells in different routes: on the one hand, it promoted cell apoptosis; on the other hand, it induced cell death with other pathways.

3.2 Cell cycle detection

Most cells of control and treatment groups maintained in G0/G1 phase. 48 h after Compound (2) (40μg/mL) treatment, percentage of cells in G0/G phase decreased. 48 h after Compound (2) (80μg/mL) treatment, percentage of G0/G phase cells decreased, compared with that of the Control group, and half of cells stayed in S phase and G2/M phase. With the increasing of the concentration and administration time, cells in G0/G1 phase decreased gradually, but cells in S phase increased. For example, when cells were treated with Compound (2) at the concentration of 80 μg/mL for 24 h, 48h and 72 h respectively, the percentage of cells in S phase increased to (20.39±2.56) %, (30.95±5.84) % and (40.34± 12.32) % respectively, which are higher than that of the Control group. The results above indicated that Compound (2) retardant cells in S phase, inhibited cells entered into G0/G1 phase and inhibited proliferation of tumor cells with dose-dependent manner.

Table 3 Effect of Compound (2) to HepG2 cell cycle

| Group | dosage (μg/mL) | 24h | | | 48h | | | 72h | | |
|---|---|---|---|---|---|---|---|---|---|---|
| | | G0/G1 | S | G2/M | G0/G1 | S | G2/M | G0/G1 | S | G2/M |
| Control | - | 61.39±0.28 | 16.63±1.32 | 21.99±1.61 | 56.39±0.69 | 23.68±1.61 | 19.94±0.93 | 56.38±0.50 | 26.09±0.26 | 17.53±0.19 |
| | 20 | 67.26±1.13 | 17.22±0.12 | 15.57±0.97 | 60.62±0.49 | 16.11±4.51 | 23.27±6.00 | 59.77±3.52 | 26.27±5.57 | 13.96±2.04 |
| Compound (2) | 40 | 52.52±5.79 | 17.53±0.12 | 29.95±5.67 | 55.08±5.75 | 12.29±6.02 | 32.64±1.77 | 63.09±6.12 | 16.48±14.52 | 20.44±20.44 |
| | 80 | 55.19±1.85 | 20.39±2.56 | 21.20±7.05 | 47.28±13.07 | 30.95±5.84 | 21.77±7.22 | 49.28±10.68 | 40.34±12.32 | 10.39±1.64 |

**Example 6**

**Effects of *Wacao* saponins on anti-HepG2 human heptoma in BALB/c (nu/nu) nude mice**

This experiment is to investigate the effect of Compound (1) or (2) on anti-HepG2 human heptoma in BALB/c (nu/nu) nude mice.

**Animals and Breeding**

70 male BALB/c (nu/nu) nude mice (18-20g, License NO. SCXK (BJ) 2009-0017, National Institutes for Food and Drug Control.) were adaptively bred for one week, and then used in the following experiment.

Animals were housed under the following condition, as shown in Table 4

Table 4 The breeding conditions

| | |
|---|---|
| Temperature | 22°C±2°C |
| lighting | 12hs light- dark cycle |
| A 12h light- dark cycle means the alternate time of light and dark for 12hs respectively. | |

Animals were housed individually in wood-chip-bedded plastic cages and fed the standard laboratory chow. All the animals had *ad libitum* access to food and water.

**HepG2 cell line culture**

Human resources hepatoma cell line HepG2 was cultured in RPMI-1640 medium containing 100U/mL Penicillin and 100μg/mL Streptomycin with 10 % fetal bovine serum and maintained at 37 °C in a humidified atmosphere(5 % $CO_2$ and 95% $O_2$),

**Experimental Design**

70 BALB/c (nu/nu) nude mice were adaptively bred for 1 week, HepG2 cells were inoculated subcutaneously into the back of nude mice, $1\times10^7$ cells per mouse. Mice were randomly assigned into 7 groups: Model group, *Wacao* total saponins (prepared with the method of example 1) group, Compound (1) group, Compound (2) group, Compound (1)+ Cytoxan group, Compound (2)+ Cytoxan group and Cytoxan group as the positive one, 10 mice per group. 3 days after inoculation, mice of every therapeutic group were all given the relevant compounds or drugs for 3 weeks, and others were given isodose physiological saline.

The body weight and the size of tumor were measured and recorded weekly. The tumor volume was calculated with the formula below:
tumor volume $V=\pi/6\times a\times b^2$ (a, b means the transverse and longitudinal length of tumor)

At the end of administration, mice were fastened for 8 hours, weighed the fasting body weight, dropped off eyeball to collect blood, centrifuged and collected serum for next experiment. The tumors were departed and weighed. Tumor Index and inhibition rate were calculated as below:

$$\text{Tumor Index} = \text{Tumor mass (mg)/body weight of animal (g)}$$

$$\text{Inhibition rate} = (\text{Average tumor mass}_{\text{Control group}} - \text{Average tumor mass}_{\text{administration group}})/\ \text{Average tumor mass}_{\text{Control group}}\times 100\ \%$$

Animals were treated as follows:

Table 5 The administration and dosage of animals

| Groups | Drugs | Dosage (mg/kg BW ) |
|---|---|---|
| Model group | sterile water | - |

(continued)

| Groups | Drugs | Dosage (mg/kg BW ) |
|---|---|---|
| *Wacao* total saponins group | *Wacao* total saponins | 1.5 |
| Compound (1) group | Compound (1) | 1 |
| Compound (2) group | Compound (2) | 1 |
| Compound (1)+ Cytoxan group | Compound (1) & Cytoxan | 1&39 |
| Compound (2)+ Cytoxan group | Compound (2) & Cytoxan | 1&39 |
| Cytoxan group | Cytoxan | 40 |

**Medication**

*Wacao* total saponins group, Compound (1), (2) and Cytoxan were respectively dissolved into sterile water, sterilized with filtration. Compound (1) + Cytoxan, Compound (2) + Cytoxan were prepared as 1:39 respectively, sterilized with filtration. Mice in the model group were injected with sterile water (10 ml/kg BW), Sc. All the animals were treated at 9 am for 3 weeks.

**Experimental Procedures**

Determination of body weights: Body weights of animals were weighed and recorded once a week.

Tumor measurement: The transverse and longitudinal lengths of tumor were measured with vernier caliper weekly.

End of the experiment : After mice being administrated for 3 weeks (21 days), the experiment was terminated.

**Statistical Analysis**

Results were expressed as means ± SD. Data analyses were performed using analysis of variance with the SPSS 10.0 software for Windows.

**Results**

Effects of *Wacao* saponins on body weight in BALB/c (nu/nu) nude mice

Before administration, the average body weight of every group is similar with no statistical difference among groups. After inoculated HepG2 cells, most body weights of mice increased, especially the body weights of Compound (2) group grew faster than that of the Model group. By contrast, the body weights of Cytoxan group decreased much more than that of the Model group (p<0.01). These results indicated that *Wacao* total saponins, Compound (1) or (2) ameliorated the decrease of body weight caused by tumor or Chemotherapeutic drugs. The slowly increase of body weight of combined drug groups further indicated that Compound (1) or (2) can obviously reduce the toxic and side effects of CY, as shown in Table 6.

Table 6 The influence of different *Wacao* saponins to the body weight of HepG2-bearing nude mice

| Group | Administration time | | | |
|---|---|---|---|---|
| | 0W | 1W | 2W | 3W |
| Model | 19.20±0.63 | 20.30±0.82 | 20.60±1.17 | 20.80±1.14 |
| *Wacao* total saponins | 19.60±0.42 | 21.20±0.55 | 21.50±2.25 | 22.60±1.64** |
| Compound (1) | 19.40±0.84 | 21.20±0.79* | 22.10±0.88** | 22.40±0.84** |
| Compound (2) | 19.50±0.53 | 21.60±0.84** | 22.40±1.17** | 22.70±0.95** |
| Compound (1)+ Cytoxan | 19.30±0.81 | 20.40±1.22 | 21.30±1.63 | 21.90±1.82 |
| Compound (2)+ Cytoxan | 19.40±0.77 | 20.50±1.58 | 20.50±1.82 | 21.10±1.08 |

(continued)

| Group | Administration time | | | |
|---|---|---|---|---|
| | 0W | 1W | 2W | 3W |
| Cytoxan | 19.20±0.63 | 18.50±1.08** | 16.00±1.41** | 14.70±1.34** |
| Note: *$p$<0.05, **$p$<0.01, *vs* model group | | | | |

Effects of *Wacao* saponins on tumor volume in HepG2-bearing BALB/c (nu/nu) nude mice

During the experimental period, the tumor size increased with time. The tumor size of model group grew fast, but compounds of *Wacao* saponins groups and Cytoxan group grew obviously slower than that of the Model group.

At different experimental period, all of the tumor sizes of administrating groups decreased obviously than that of the Model group (p<0.01). When the drugs were used alone, the tumor volume of Compound (2) group is the smallest, followed by Compound (1). Tumor volume of these two groups was significantly smaller than that of the Cytoxan group, which indicated that the anti-tumor activity of Compound (1) or (2) is much better than Cytoxan. Result also showed that the *Wacao* total saponins had stronger inhibitory effects on tumor growth, but such effects were weaker than that of the Cytoxan.

When mice were administrated Compound (1) or (2) combined with CY, the tumor growth was inhibited more obviously, and the tumor volume decreased much more than that of the single drug group. Data showed that Compound (1) or (2) combined with CY has better synergistic anti-tumor activity, as shown in Table 7.

Table 7 The influence of different *Wacao* saponins to the tumor volume of HepG2-bearing nude mice

| Groups | Administration time | | |
|---|---|---|---|
| | 1W | 2W | 3W |
| Model | 1800.38±645.60 | 2648.61±990.41 | 4297.36±1471.43 |
| *Wacao* total saponins | 1385.43±456.82* | 1625.71±515.46** | 2298.50±578.24** |
| Compound (1) | 922.51±624.95** | 1060.32±570.38** | 1033.33±656.54** |
| Compound (2) | 564.84±474.95** | 841.36±577.72** | 911.76±460.22** |
| Compound (1)+ Cytoxan | 743.14±321.30** | 894.02±320.85** | 953.97±540.38** |
| Compound (2)+ Cytoxan | 432.09±125.44** | 745.35±301.33** | 798.30±235.77** |
| Cytoxan | 988.47±705.60** | 1220.93±881.80** | 1593.79±955.17** |
| Note: *$p$<0.05, **$p$<0.01, *vs* model group | | | |

Effects of different *Wacao* saponins on the tumor mass, tumor index and inhibition rate of HepG2-bearing nude mice

*Wacao* total saponins, Compound (1) or (2) showed the good inhibitory effects on tumor mass and tumor index ($p$<0.01, *vs* Model group). When the drugs were used alone, the inhibitory activity of Compound (2) to tumor growth was the best, the tumor mass and tumor index of Compound (2) group were lower than that of the Cytoxan; *Wacao* total saponins also improved the index of above, but its effect was weaker than that of the Cytoxan group. According to the tumor inhibition, the inhibition rates of Compound (1) or (2) were 80.05% and 87.42 % respectively, which were higher than that of the Cytoxan group (70.46 %).Results showed that the anti-tumor activities of Compound (1) or (2) were stronger than that of the Cytoxan.

When mice were administrated Compound (1) or (2) combined with CY, the tumor mass decreased more obviously, and the tumor index and inhibition rate increased. Data showed that Compound (1) or (2) combined with CY has better synergistic anti-tumor activity, as shown in Table 8.

Table 8 The influence of different *Wacao* saponins to the tumor mass and index of HepG2-bearing nude mice

| Group | Tumor mass (mg) | Tumor index | Inhibition rate(%) |
|---|---|---|---|
| Model | 2.95±1.01 | 0.14±0.05 | - |
| *Wacao* total saponins | 1.05±0.53** | 0.07±0.02** | 62.38 |

(continued)

| Group | Tumor mass (mg) | Tumor index | Inhibition rate(%) |
|---|---|---|---|
| Compound (1) | 0.59±0.29** | 0.03±0.01** | 80.05 |
| Compound (2) | 0.37±0.33** | 0.02±0.01** | 87.32 |
| Compound (1)+Cytoxan | 0.41±0.18** | 0.02±0.01** | 85.73 |
| Compound (2)+Cytoxan | 0.30±0.15** | 0.02±0.01** | 90.14 |
| Cytoxan | 0.87±0.38** | 0.06±0.02** | 70.46 |
| Note: $^{*}p$<0.05, $^{**}p$<0.01. *vs* model group. | | | |

**Conclusions**

*Wacao* total saponins, Compound (1) or (2) could significantly increase body weight of HepG2-bearing nude mice, decrease tumor volume, mass and tumor index. The inhibition rates of Compound (1) or (2) were higher than that of the Cytoxan, which indicated that *Wacao* saponins have stronger anti-tumor activity. At the same time, Compound (1) or (2) increased the body weight of HepG2-bearing nude mice (p<0.05 or p<0.01, *vs* model group), which indicated that the side effect of *Wacao* saponins is weaker than that of the Cytoxan. Therefore, *Wacao* saponins of Compound (1) or (2) can be used as a high efficiency and low toxicity ingredient to treat tumor. When treated with Compound (1) or (2) combined with Cytoxan, body weights of mice and inhibition rates of anti-tumor were increased, which indicated that Compound (1) or (2) can decrease toxicity and increase efficacy when used with Cytoxan.

**Example 7**

**Effects of *Wacao* saponins on anti-BGC823 human gastric adenocarcinoma in BALB/c (nu/nu) nude mice**

**Animals and Breeding**

70 male BALB/c (nu/nu) nude mice (18-20g, License NO. SCXK (BJ) 2009-0017, National Institutes for Food and Drug Control.) were adaptively bred for one week, and then used in the following experiment.

Experimental animals were housed in the same condition as the experiment 4.

**BGC823 cell line culture**

Human resources gastric adenocarcinoma cell line BGC823 was cultured in the same condition as the experiment 4.

**Experimental Design**

70 BALB/c (nu/nu) nude mice were adaptively bred for 1 week. BGC823 cells were inoculated subcutaneously into the back of nude mice, $1.2\times10^7$ cells per mouse. Mice were randomly assigned into 7 groups: Model group, *Wacao* total saponins (prepared according to the method of example 1) group, Compound (1) group, Compound (2) group, Compound (1)+ 5-FU group, Compound (2)+ 5-FU group and 5-FU group as the positive one, 10 mice per group. 3 days after inoculation, the mice of every therapeutic group were all given the relevant compounds or drugs for 3 weeks, and others were given isodose physiological saline.

The body weight and the tumor volume were measured and recorded weekly. The tumor volume was calculated according to the formula below.

Tumor volume $V=\pi/6\times a\times b^2$ (a, b means the transverse and longitudinal length of tumor)

At the end of administration, mice were fastened for 8 hours, weighed the fasting body weight, dropped off eyeball to collect blood, centrifuged and collected serum for further experiment. Tumors were departed and weighed. Tumor Index and inhibition rate were calculated as below:

$$\text{Tumor Index} = \text{Tumor mass (mg)/body weight of animal (g)}$$

$$\text{Inhibition rate} = (\text{Average tumor mass}_{\text{Control group}} - \text{Average tumor mass}_{\text{administration group}}) / \text{Average tumor mass}_{\text{Control group}} \times 100\ \%$$

Animals were treated as follows:

Table 5 The administration and dosage of animals

| Groups | Drugs | Dosage (mg/kg BW ) |
|---|---|---|
| Model group | sterile water | - |
| *Wacao* total saponins group | *Wacao* total saponins | 1.5 |
| Compound (1) group | Compound (1) | 1 |
| Compound (2) group | Compound (2) | 1 |
| Compound (1)+5-FU group | Compound (1) &5-FU | 1&19 |
| Compound (2)+5-FU group | Compound (2) & 5-FU | 1&19 |
| 5-FU group | 5-FU | 20 |

**Medication**

*Wacao* total saponins group, Compound (1), (2) and 5-FU were respectively dissolved into sterile water, sterilized with filtration. Compound (1) + 5-FU, Compound (2) + 5-FU were prepared as 1:19 respectively, sterilized with filtration. Mice in the model group were injected with sterile water (10 ml/kg BW), Sc. All the animals were treated at 9 am for 3 weeks.

**Experimental Procedures**

Determination of body weights: Body weights of animals were weighed and recorded once a week.
Tumor measurement: The transverse and longitudinal lengths of tumor were measured weekly.
End of the experiment : After mice being administrated for 3 weeks (21 days), the experiment was terminated.

**Statistical Analysis**

Results were expressed as means ± SD. Data analyses were performed using analysis of variance with the SPSS 10.0 software for Windows.

**Results**

Effect of *Wacao* saponins on body weight in BGC823-bearing BALB/c (nu/nu) nude mice

Before administration, the average body weight of every group is similar with no statistical difference among groups. After inoculated BGC823 cells, the body weight of model group grew very slow, and decreased significantly in the 3$^{rd}$ week; the body weight of *Wacao* total saponins group, Compound (1) or (2) group increased every week, with no difference among 3 groups. The average body weight of 5-FU group was smaller than that of the Model group ($p<0.01$). The slowly increase of body weight of combined drug groups further indicated that Compound (1) or (2) can obviously reduce the toxic and side effects of 5-FU. The results indicated that Compound (1) or (2) ameliorated the decrease of body weight caused by 5-FU, as shown in Table 10.

Table 10 The influence of different *Wacao* saponins to the body weight of BGC823-bearing nude mice

| Group | Administration time | | | |
|---|---|---|---|---|
| | 0W | 1W | 2W | 3W |
| Model | 20.40±1.26 | 21.60±1.17 | 21.40±1.17 | 18.50±1.08 |
| *Wacao* total saponins | 20.50±1.42 | 21.80±1.33 | 22.40±1.43* | 23.50±1.34** |

(continued)

| Group | Administration time | | | |
|---|---|---|---|---|
| | 0W | 1W | 2W | 3W |
| Compound (1) | 20.30±1.06 | 21.50±1.18 | 22.70± 1.16* | 23.70±1.06** |
| Compound (2) | 20.10±1.10 | 21.90±0.88 | 23.00±0.82** | 23.40±1.58** |
| Compound (1)+5-FU | 20.40±1.42 | 20.70±1.33 | 21.60±1.63 | 22.5±1.17 |
| Compound (2)+5-FU | 20.50±1.28 | 21.10±1.59 | 21.80±1.52 | 22.40±2.79 |
| 5-FU | 20.20±1.23 | 19.50±1.51** | 18.20±1.81** | 16.70±2.20** |
| Note: *$p$<0.05, **$p$<0.01, *vs* model group | | | | |

Effect of *Wacao* saponins on tumor volume in BALB/c (nu/nu) nude mice

In the first week, the tumor size of each treatment group decreased obviously and was smaller than that of the Model group. The decrease showed much more significant in Compound (1) or (2) groups. *Wacao* total saponins also showed certain anti-BGC823 effect, but it is weaker than 5-FU.

When mice were administrated Compound (1) or (2) combined with 5-FU, the tumor growth was inhibited more obviously than that of the single drug group. So to speak, Compound (1) or (2) combined with 5-FU has better synergistic anti-tumor activity, as shown in Table 11.

Table 11 The influence of different *Wacao* saponins to the tumor volume of BGC823-bearing nude mice

| Groups | Administration time | | |
|---|---|---|---|
| | 1W | 2W | 3W |
| Model | 1635.75±555.82 | 2382.79±816.07 | 4140.42±1462.44 |
| *Wacao* total saponins | 964.28±575.29** | 1413.49±685.13** | 2756.42±885.76** |
| Compound (1) | 850.16±539.70** | 997.88±499.61** | 845.04±239.21** |
| Compound (2) | 796.43±563.35** | 1186.34±625.68** | 1654.23±891.57** |
| Compound (1)+5-FU | 732.23±255.13** | 743.27±264.92** | 673.29±251.15** |
| Compound (2)+5-FU | 644.39±274.82** | 733.90±283.73** | 1025.38±533.87** |
| 5-FU | 931.38±623.32** | 1262.33±764.46** | 2212.36±1196.96** |
| Note: *$p$<0.05, **$p$<0.01, *vs* model group | | | |

Effect of different *Wacao* saponins on the tumor mass, tumor index and inhibition rate of BGC823-bearing nude mice

Results showed that *Wacao* total saponins, Compound (1) or (2) could decrease the tumor mass and tumor index ($p$<0.01, *vs* Model group), and the effect of Compound (2) is better than that of the Compound (1) and *Wacao* total saponins. When the drugs were used alone, the inhibition rates of Compound (1) or (2) were 64.54% and 79.63 % respectively, which were higher than that of the 5-FU and *Wacao* saponins. Therefore, *Wacao* saponins especially Compound (2) showed a very strong anti-tumor activity.

When mice were administrated Compound (1) or (2) combined with 5-FU, tumor mass and tumor index decreased and the inhibition rate increased more obviously. Hence Compound (1) or (2) combined with 5-FU has better synergistic anti-tumor activity, as shown in Table 12.

Table 12 The influence of different *Wacao* saponins to tumor mass and index of BGC823-bearing nude mice

| Group | Tumor mass (mg) | Tumor index | Inhibition rate(%) |
|---|---|---|---|
| Model | 2.72±0.73 | 0.15±0.05 | - |
| *Wacao* total saponins | 1.41±0.47** | 0.10±0.02** | 51.20 |

(continued)

| Group | Tumor mass (mg) | Tumor index | Inhibition rate(%) |
|---|---|---|---|
| Compound (1) | 1.05±0.36** | 0.04±0.02** | 61.54 |
| Compound (2) | 0.55±0.20** | 0.02±0.01** | 79.63 |
| Compound (1)+5-FU | 0.73±0.22** | 0.03±0.01** | 70.27 |
| Compound (2)+5-FU | 0.34±0.20** | 0.02±0.01** | 85.39 |
| 5-FU | 1.23±0.53** | 0.08±0.04** | 54.85 |
| Note: *$p$<0.05, **$p$<0.01. *vs* model group. | | | |

**Conclusions**

Results showed that *Wacao* total saponins, Compound (1) or (2) could significantly increase body weights of BGC823-bearing nude mice, decrease tumor volume, mass and tumor index, and increase the inhibition rate. Especially the inhibition rates of Compound (1) or (2) were higher than that of the 5-FU, which indicated that *Wacao* saponins have stronger anti-tumor activity. At the same time, Compound (1) or (2) increased the body weight of BGC823-bearing nude mice ($p$<0.05 or $p$<0.01, *vs* model group), which indicated that the side effect of *Wacao* saponins is weaker than that of the 5-FU, and can be used as a high efficiency and low toxicity ingredient to treat tumor. When Compound (1) or (2) were administrated combined with 5-FU, the body weights of mice and inhibition rates of anti-tumor were increased, which indicated that Compound (1) or (2) could decrease toxicity and increase efficacy when combined with 5-FU.

**Example 8**

**Effects of Wacao saponins on anti-HT29 human Colon cancer in BALB/c (nu/nu) nude mice**

**Animals and Breeding**

70 male BALB/c (nu/nu) nude mice (18-20g, License NO. SCXK (BJ) 2009-0017, National Institutes for Food and Drug Control.) were adaptively bred for one week, and then used in the following experiment.

Experimental animals were housed in the same condition as the experiment 4.

**HT29 cell line culture**

Human resources Colon cancer cell line HT29 was cultured in the same condition as the experiment 4.

**Experimental Design**

70 BALB/c (nu/nu) nude mice were adaptively bred for 1 week, HT29 cells were inoculated subcutaneously into the back of nude mice, $1.0\times10^7$ cells per mouse. Mice were randomly assigned into 7 groups: Model group, *Wacao* total saponins (prepared according to the method of example 1) group, Compound (1) group, Compound (2) group, Compound (1)+ Paclitaxel (PTX) group, Compound (2)+ PTX group and PTXgroup as the positive one, 10 mice per group. 3 days after inoculation, the mice of every therapeutic group were all given the relevant compounds or drugs for 3 weeks, and others were given isodose physiological saline.

The body weight and the tumor volume were measured and recorded weekly. The tumor volume was calculated according to the formula below.

tumor volume $V=\pi/6\times a\times b^2$ (a,b means the transverse and longitudinal length of tumor)

At the end of administration, mice were fastened for 8 hours, weighed the fasting body weight, dropped off eyeball to collect blood, centrifuged and collected serum for further experiment. The tumors were departed and weighed. Tumor Index and inhibition rate were calculated as below:

$$\text{Tumor Index} = \text{Tumor mass (mg)/body weight of animal (g)}$$

$$\text{Inhibition rate} = (\text{Average tumor mass}_{\text{Control group}} - \text{Average tumor mass}_{\text{administration group}})/\text{Average tumor mass}_{\text{Control group}} \times 100\ \%$$

Animals were treated as follows:

Table 13 The administration and dosage of animals

| Groups | Drugs | Dosage (mg/kg BW ) |
|---|---|---|
| Model group | sterile water | - |
| *Wacao* total saponins group | *Wacao* total saponins | 1.5 |
| Compound (1) group | Compound (1) | 1 |
| Compound (2) group | Compound (2) | 1 |
| Compound (1)+ PTX group | Compound (1) & PTX | 1&19 |
| Compound (2)+ PTX group | Compound (2) & PTX | 1&19 |
| PTX group | PTX | 20 |

**Medication**

*Wacao* total saponins group, Compound (1), (2) and PTX were respectively dissolved into sterile water, terilized with filtration. Compound (1) + PTX, Compound (2) +PTX were prepared as 1:19 respectively, sterilized with filtration. Mice in the model group were injected with sterile water (10 ml/kg BW), Sc. All the animals were treated at 9 am for 3 weeks.

**Experimental Procedures**

Determination of body weights: Body weights of animals were weighed and recorded once a week.
Tumor measurement: The transverse and longitudinal lengths of tumor were measured weekly.
End of the experiment : After mice being administrated for 3 weeks (21 days), the experiment was terminated.

**Statistical Analysis**

Results were expressed as means ± SD. Data analyses were performed using analysis of variance with the SPSS 10.0 software for Windows.

**Results**

Effect of *Wacao* saponins on body weight in HT29-bearing BALB/c (nu/nu) nude mice

Before administration, the average body weight of every group is similar with no statistical difference among groups. After inoculated HT29 cells for 1 week, the body weights of all groups increased except for PTX group of which body weight decreased a little bit. 2 weeks after inoculation, the body weight of *Wacao* total saponins group, Compound (1) or (2) groups kept increasing, but that of the Model group and PTX group began to decrease. In the 3$^{rd}$ week, the body weights of all groups except for Compound (1) group decreased, especially the model and PTX groups decreased much more.
When mice were administrated with Compound (1) or (2) combined with PTX, the body weight increased slowly, which indicated that Compound (1) or (2) can obviously reduce the toxic and side effects of PTX. The results indicated that Compound (1) or (2) ameliorated the decrease of body weight caused by PTX, as shown in Table 14.

Table 14 The influence of different *Wacao* saponins to the body weight of HT29-bearing nude mice

| Group | Administration time | | | |
|---|---|---|---|---|
| | 0W | 1W | 2W | 3W |
| Model | 20.40±1.51 | 21.90±1.35 | 21.00±1.15 | 18.40±0.98 |

(continued)

| Group | Administration time | | | |
|---|---|---|---|---|
| | 0W | 1W | 2W | 3W |
| *Wacao* total saponins | 20.30±1.66 | 22.40±0.85 | 23.20±0.84** | 22.50±1.05** |
| Compound (1) | 20.40±1.27 | 21.90±0.90 | 23.00±1.15** | 24.10±0.90** |
| Compound (2) | 20.10±1.35 | 22.10±0.90 | 22.90±0.90** | 22.70±1.38** |
| Compound (1)+PTX | 20.30±1.53 | 21.60±1.22 | 22.40±1.59** | 23.40±1.82** |
| Compound (2)+PTX | 20.40±1.64 | 21.40±1.56 | 22.70±1.65** | 22.90±1.77** |
| PTX | 20.60±1.32 | 20.30±1.68* | 19.80±1.77** | 19.20±1.37** |
| Note: *$p$<0.05, **$p$<0.01, *vs* model group | | | | |

Effect of *Wacao* saponins on tumor volume in HT29-bearing BALB/c (nu/nu) nude mice

In the first week, the tumor size of each drug treatment group was smaller than that of the Model group, but no statistical difference was found when compared with model group. In the second and the third week, tumor size of Compound (2) and PTX groups increased first and then decreased. The tumor size of Compound (1) group showed continuously decrease, which is much smaller compared with model group ($p$<0.05 or $p$<0.01).

When mice were administrated Compound (1) or (2) combined with PTX, the tumor growth was inhibited more obviously than that of the single drug group. So to speak, Compound (1) or (2) combined with PTX has better synergistic anti-tumor activity, as shown in Table 15.

Table 15 The influence of different *Wacao* saponins to the tumor volume of HT29-bearing nude mice

| Groups | Administration time | | |
|---|---|---|---|
| | 1W | 2W | 3W |
| Model | 1767.38±604.82 | 2247.67±1021.95 | 2744.03±742.64 |
| *Wacao* total saponins | 1225.37±576.35 | 1511.66±801.63* | 1879.68±875.05** |
| Compound (1) | 1094.99±678.00 | 907.89±738.11* | 814.93±450.73** |
| Compound (2) | 639.16±539.16** | 918.54±469.16* | 859.24±691.16** |
| Compound (1)+ PTX | 855.36±322.81** | 843.92±339.60** | 712.81±225.49** |
| Compound (2)+ PTX | 542.38±231.83** | 755.30±232.44* | 682.93±310.52** |
| PTX | 943.55±516.62** | 996.66±347.05* | 801.38±259.34** |
| Note: *$p$<0.05, **$p$<0.01, *vs* model group | | | |

The influence of different *Wacao* saponins on tumor mass, tumor index and inhibition rate of HT29-bearing nude mice

Results showed that *Wacao* total saponins, Compound (1) or (2) could decrease tumor mass and tumor index ($p$<0.01, *vs* Model group), and the effect of Compound (1) or (2) is similar to that of the PTX. When the drugs were used alone, the inhibition rates of Compound (1) or (2) were 50.44% and 54.87 % respectively, and total saponins was 46.76%, which was lower than that of the Compound (1) or (2).

When mice were administrated Compound (1) or (2) combined with PTX, the tumor mass and tumor index decreased and the inhibition rates increased more obviously than that of the single drug. Hence, Compound (1) or (2) combined with PTX has better synergistic anti-tumor activity, as shown in Table 16.

Table 16 The influence of different *Wacao* saponins to the tumor mass and index of HT29-bearing nude mice

| Group | Tumor mass (mg) | Tumor index | Inhibition rate(%) |
|---|---|---|---|
| Model | 2.32±0.50 | 0.13±0.03 | - |

(continued)

| Group | Tumor mass (mg) | Tumor index | Inhibition rate(%) |
|---|---|---|---|
| *Wacao* total saponins | 1.23±0.34** | 0.06±0.02** | 46.76 |
| Compound (1) | 1.15±0.52** | 0.05±0.02** | 50.44 |
| Compound (2) | 1.05±0.28** | 0.05±0.01** | 54.87 |
| Compound (1)+ PTX | 0.78±0.25** | 0.04±0.02** | 71.23 |
| Compound (2)+ PTX | 0.65±0.22** | 0.03±0.01** | 75.44 |
| PTX | 0.91±0.43** | 0.06±0.03** | 60.81 |
| Note: $^{*}p$<0.05, $^{**}p$<0.01. *vs* model group. | | | |

**Conclusions**

Results showed that *Wacao* total saponins, Compound (1) or (2) could significantly increase body weight of HT29-bearing nude mice, decrease tumor volume, mass and tumor index, which showed good anti-Colon cancer activity. When Compound (1) or (2) were combined with PTX, the body weight of mice and the inhibition rate of anti-tumor increased, which indicated that Compound (1) or (2) can decrease toxicity and increase efficacy when combined with PTX.

**Example 9**

**Preparation of tablets**

The preparation process was shown as follows: 33g Compound (1) or (2) added with 217g starch were well mixed. The mixture was granulated with proper starch paste used as an adhesive. Then the granulation was dried and tablets were prepared. Adults take orally 1 tablet (250mg per tablet), twice daily.

**Example 10**

**Preparation of capsules**

The preparation process was shown as follows: 33g Compound (1) or (2) added with 217g starch were well mixed. The mixture was granulated with proper starch paste used as an adhesive. Then the granulation was dried and capsules were prepared. Adults take orally 1 capsule (250mg per capsule), twice daily.

**Example 11**

**Preparation of injection**

33g Compound (1) or (2) was dissolved in the injection water. 30g activated carbon was added into each solution (105°C activated 1h) to reach 0.01%, then heated to boil for 20min, adjusted pH value to 5.5-6.5 and regulated to isotonic solution by adding sodium chloride. The isotonic solution was finally made into injection after the hot filtration, sealing and sterilization preparation steps. The injection should be administered by intramuscular (IM) injection in the amount of 3mL, twice daily.

**Example 12**

**Preparation of Powder-injection**

33 g Compound (1) or (2) was directly freeze-dried to fine powder. Or the fine powder was made by the Compound (1) or (2) prepared injection dissolved with 62.5g mannitol and 62.5g maltose as an excipient through freeze-drying, packaging and sterilizing operation.

### Example 13

### Preparation of liposome

33g Compound (1) or (2), 40g soy lecithin and 10g cholesterol were dissolved in the mixed solution of chloroform and methanol (v:v=2:1). The organic solvent of the mixed solution was removed by rotary evaporator vacuum distillation in water bath at 40°C. After the formation of uniform lipid film on the wall of the rotary evaporator bottle, proper amount of mannitol and glucose in aqueous solution 1000 mL was added and then the lipid membrane elution and water synthetic liposomes were shaken to make the liposome initial suspension. Finally, the liposome initial suspension was put in probe type ultrasonic to make the translucent colloidal solution, filtrated by Millipore filtration, sub-packed and sterilized to produce the liposome.

### Example 14

### Preparation of drop pills

33g Compound (1) or (2) added with 33g polyethylene glycol 4000 was melted evenly by heating at 80°C and dropped at the rate of 20 drops per minute into the liquid paraffin condensate (temperature of upper part of the condensate is controlled in the range of 5 to 15°C).The dropping pills were taken out and the condensation agent was removed. Finally 1000 pills were dried to obtain the final products with 0.0495g / pill.

### Example 15

### Sustained and controlled release preparations

**I. Matrix Tablet preparations:** 66g Compound (1) or (2) added with 40g HPMCK100M, 120g lactose and 40g starch were well mixed. The mixture was granulated with proper 5% PVP and 95% ethanol as anadhesive, then dried and tableted (266mg each). Adults take orally 1 tablet, once daily.

### II. The controlled release Preparations:

Preparation of tablet core: 33g Compound (1) or (2) added with 140g lactose, 50g starch, 3g sodium chloride and 1g talcum powder were well mixed. The mixture was granulated with proper 5%PVP and 95% ethanol as an adhesive, dried and made into tablet core (266mg each).

Preparation of coating solution: Cellulose acetate was dissolved with mixed solution of acetone and isopropanol to prepare mixed solution (2%, g/100mL). The mixed solution was added with 25% dibutyl phthalate as plasticizer and PEG400 as porogen to prepare coating solution.

Preparation of controlled release tablets: Tablet core was coated with coating solution to prepare the controlled release tablets (tablets gain 5%).

### Example 16

### Preparation of emulsion

0.5g Compound (1) or (2) added with 14mL dimethyl sulfoxide, 1.6g triethanolamine, 4.0g glycerol, 54mL distilled water, 2.0g lanolin, 10.0g white vaseline and 16.0g stearic acid were well mixed to prepare the emulsion.

### Example 17

### Preparation of buccoadhesive tablets

33g Compound (1) or (2) added with 50g HPMCK4M, 25g HPMCK15M, 85g lactose, 1.5g magnesium stearate, and 5.5g aspartame were well mixed, pulverized, and passed through 100-mesh sieve to prepare the buccoadhesive tablets (200 m g/tablet) with direct compression method. Adults take orally 1 tablet, twice daily.

**Example 18**

**Preparation of orally disintegrating tablets**

33g Compound (1) or (2) added with 146.65g low-substituted hydroxypropyl cellulose, 75g microcrystalline cellulose, 8.1g aspartame, 5.4g menthol, 35.5g lactose, 1.35g magnesium stearate were well mixed to prepare orally disintegrating tablets. Adults take orally 1 tablet, twice daily.

**Example 19**

**Preparation of granules**

33g Compound (1) or (2) added with 1167g Lactose, 2800g dextrin were well mixed. The mixture was granulated with proper 95% ethanol as an adhesive and dried to prepare granules. The granules were packed into 4g/bag. Adults take orally 1 bag, once daily.

## Claims

1. A compound of general formula I or salts, compositions containing the compound formula I or their salts for use in the treatment of tumor, wherein formula I is:

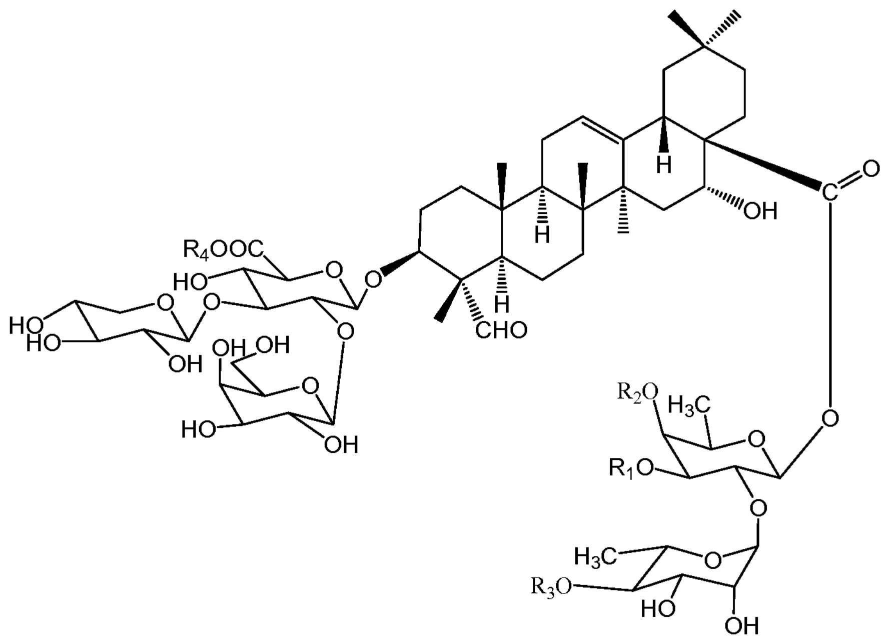

Formula I

wherein:

$R_1$=Ac;
$R_2$=(E)-MC or (Z)-MC;
$R_3$=H;
$R_4$=H, $CH_3$, or $CH_2CH_2CH_2CH_3$;

wherein Ac refers to a group as follows:

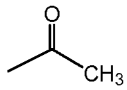

wherein (E) -MC refers to a group as follows:

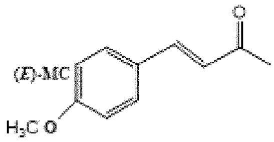

wherein (Z) -MC refers to a group as follows:

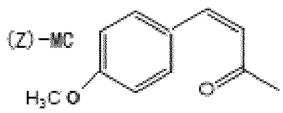

2. The compound for use of claim 1, wherein the compound (I) is:

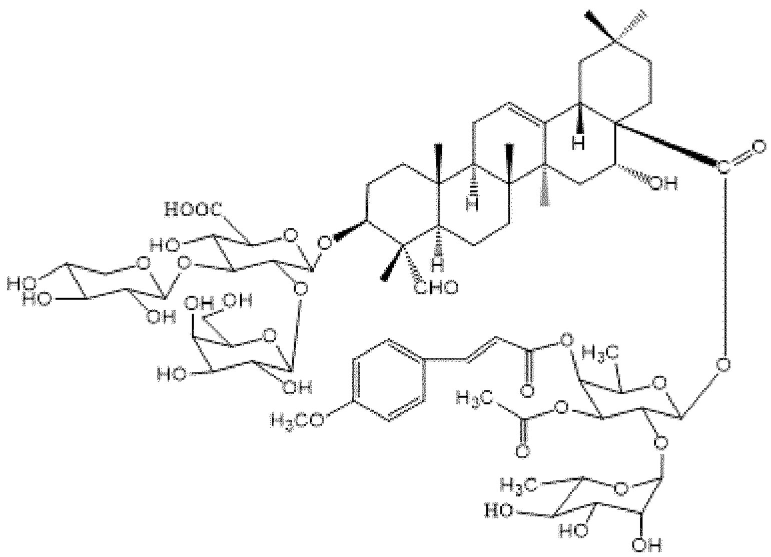

Compound (1)

Or the structural formula as follows:

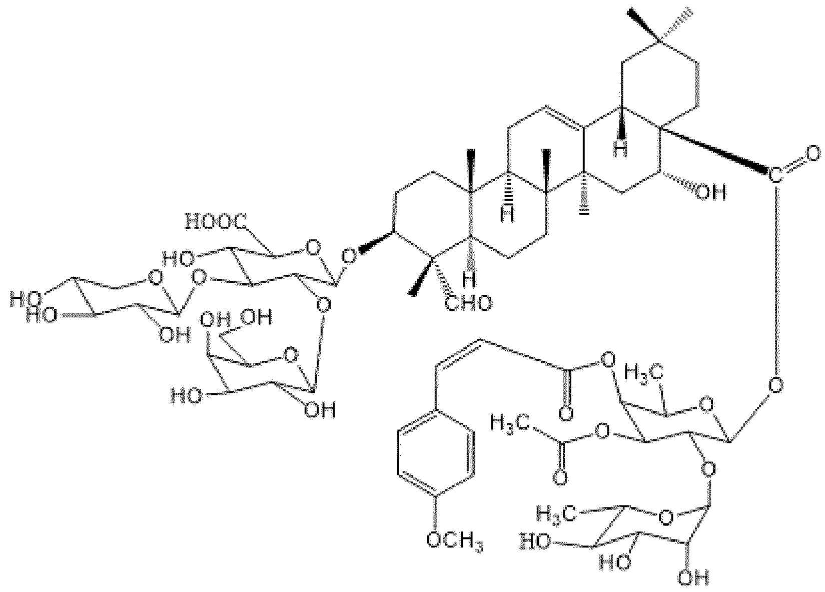

Compound (2)

Or the structural formula as follows:

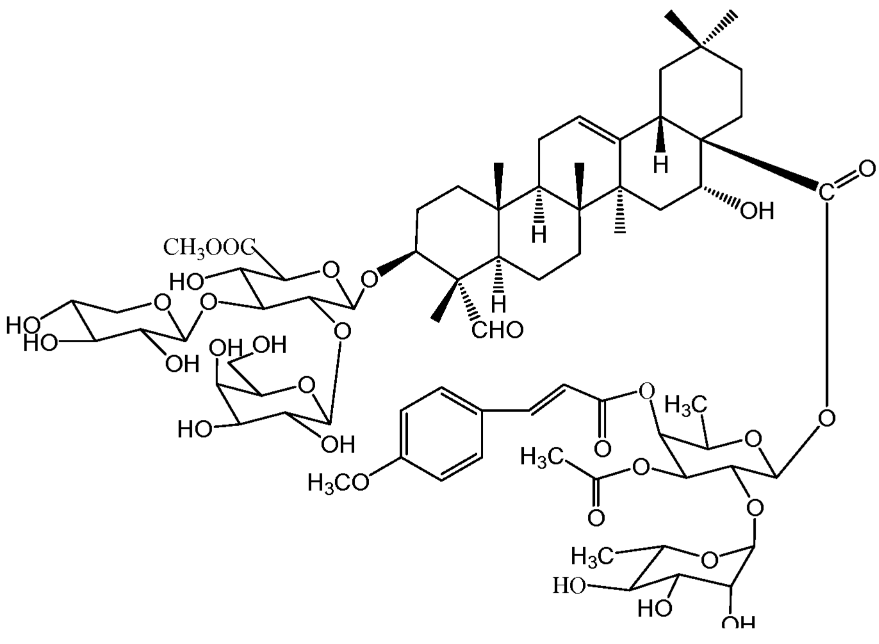

Compound (3)

Or the structural formula as follows:

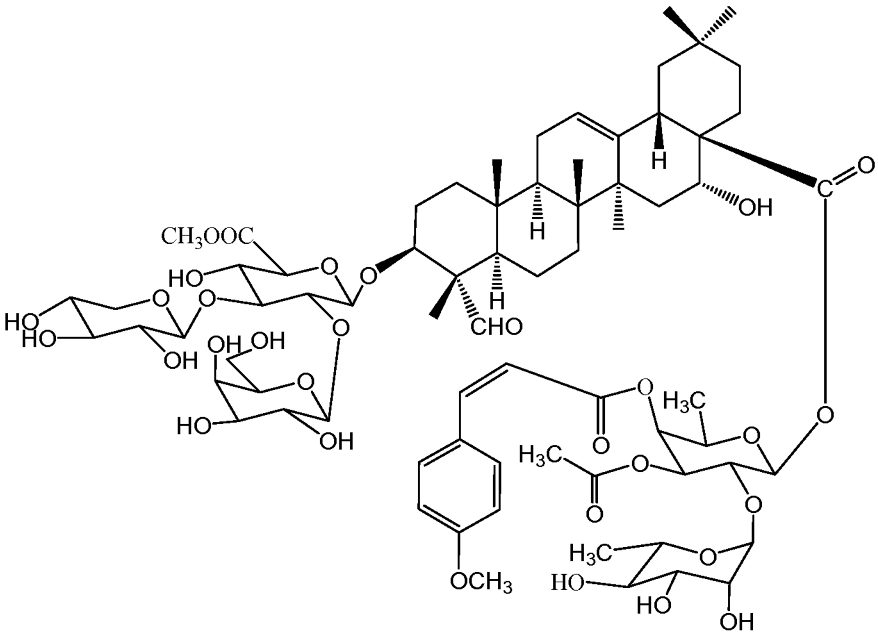

Compound (4)

Or the structural formula as follows:

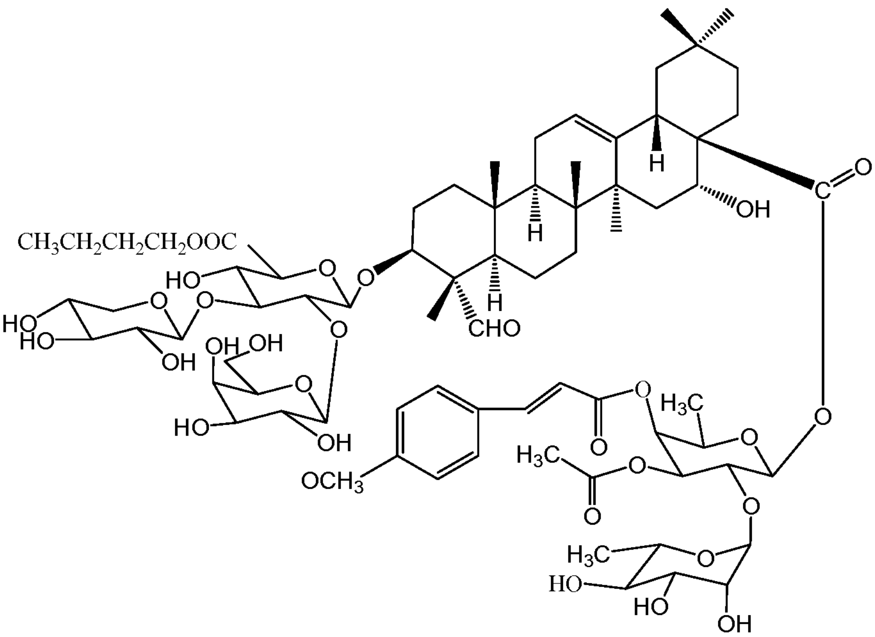

Compound (7)

Or the structural formula as follows:

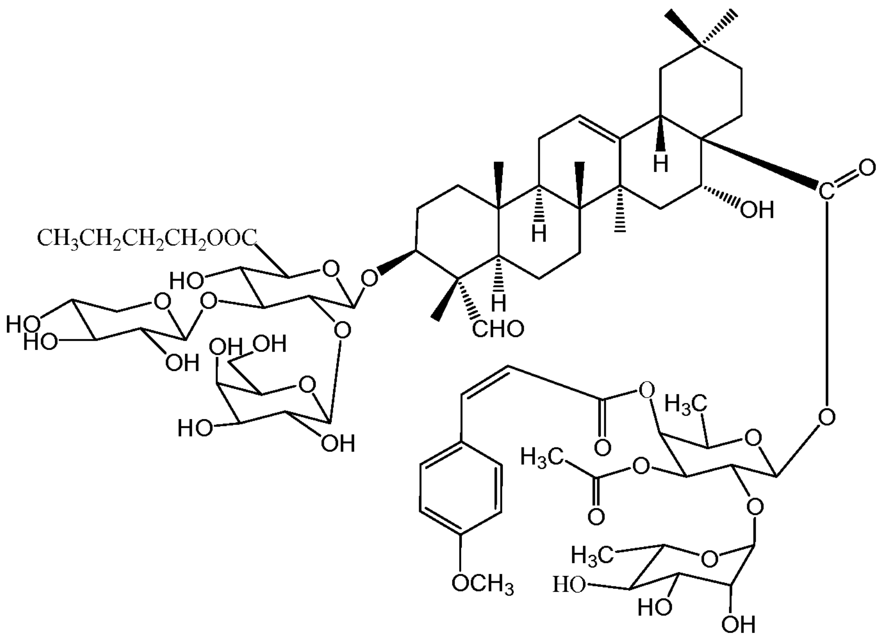

Compound (8)

3. The compound for use of claim 1, wherein the compound is used in the treatment of a cancer selected from the group consisting of liver cancer, gastric cancer, colon cancer, breast cancer and melanoma.

4. The compound for use of claim 1, wherein the salts are compounds formed by the structural formula I and alkali or alkaline earth metal ions, optionally wherein said alkalis include sodium hydroxide, potassium hydroxide, calcium hydroxide, magnesium hydroxide, sodium bicarbonate or ammonia; the said alkaline earth metal ions include sodium, potassium, calcium, aluminum, copper, zinc and magnesium.

5. The compound for use of claim 1, wherein the compound is obtained by chemosynthesis, semisynthesis or biotransformation.

6. A pharmaceutical composition comprising a compound of general formula I or salts thereof and any one of the conventional anticancer drugs, wherein formula I is:

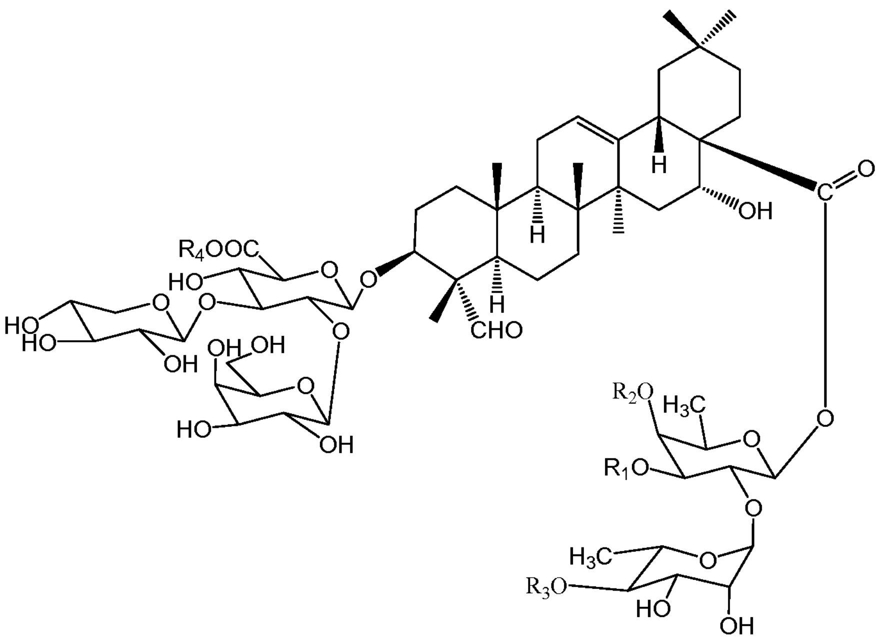

Formula I

wherein:

$R_1$= Ac;
$R_2$=(E)-MC or (Z)-MC;
$R_3$=H;
$R_4$=H, $CH_3$, or $CH_2CH_2CH_2CH_3$;

wherein Ac refers to a group as follows:

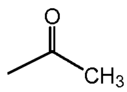

wherein (E)-MC herein defined refers to a group as follows:

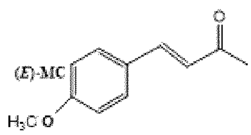

wherein (Z)-MC herein defined refers to a group as follows:

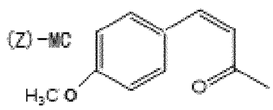

**7.** The pharmaceutical composition of claim 6, wherein the anticancer drug refers to a cyclophosphamide or 5-FU or paclitaxel.

**8.** The pharmaceutical composition of claim 6 for use in the treatment of tumor.

**9.** A pharmaceutical preparation for use in the treatment of tumor, wherein the preparation is made from compound of general formula I or salts and excipients,

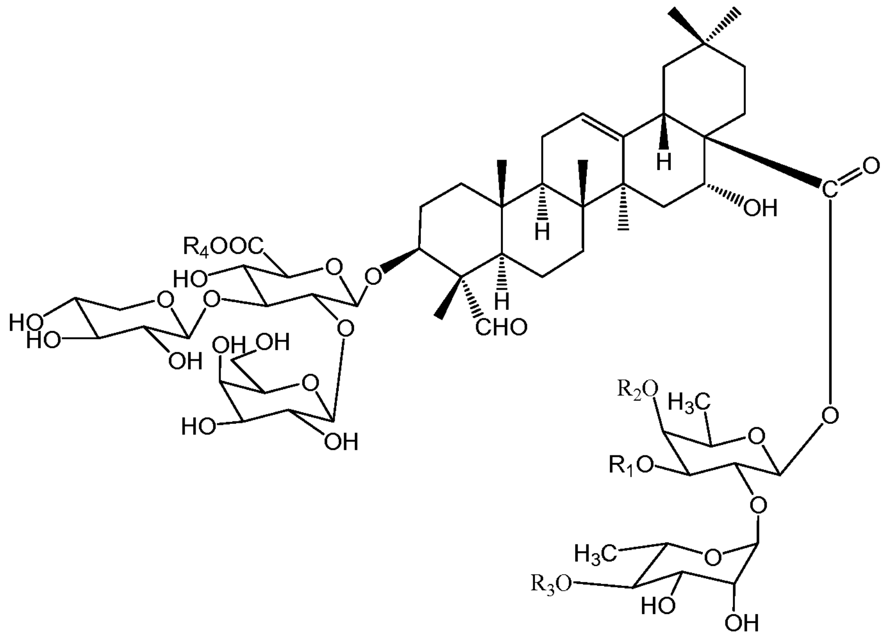

Formula I

wherein:

$R_1$= Ac;
$R_2$=(E)-MC or (Z)-MC;
$R_3$=H;
$R_4$=H, $CH_3$, or $CH_2CH_2CH_2CH_3$;

wherein Ac refers to a group as follows:

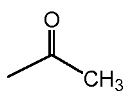

wherein (E)-MC refers to a group as follows:

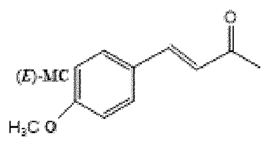

wherein (Z)-MC refers to a group as follows:

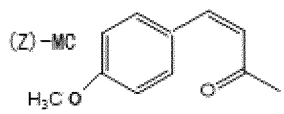

**10.** The pharmaceutical preparation for use of claim 9, wherein the compound (I) refers to a structural formula as follows:

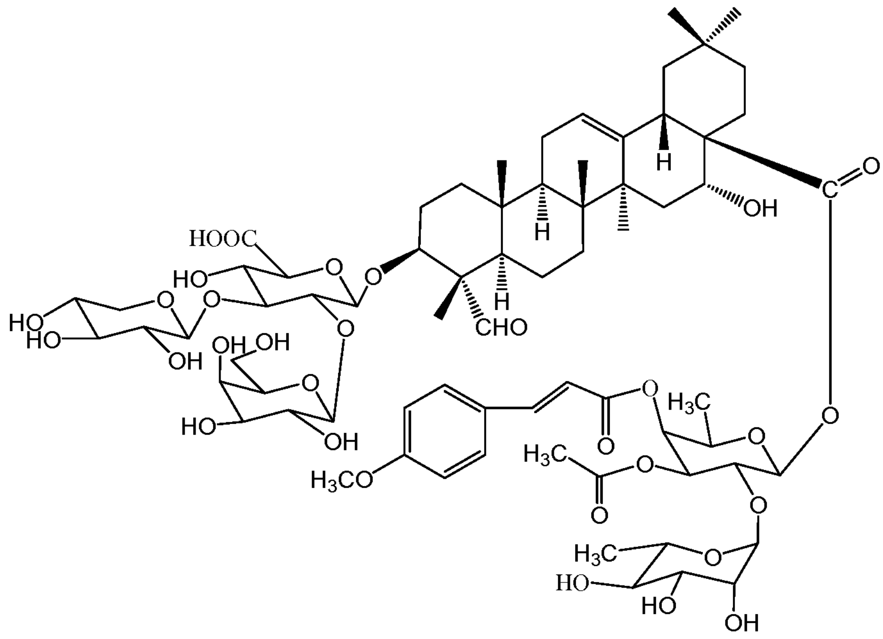

Compound (1)

Or the structural formula as follows:

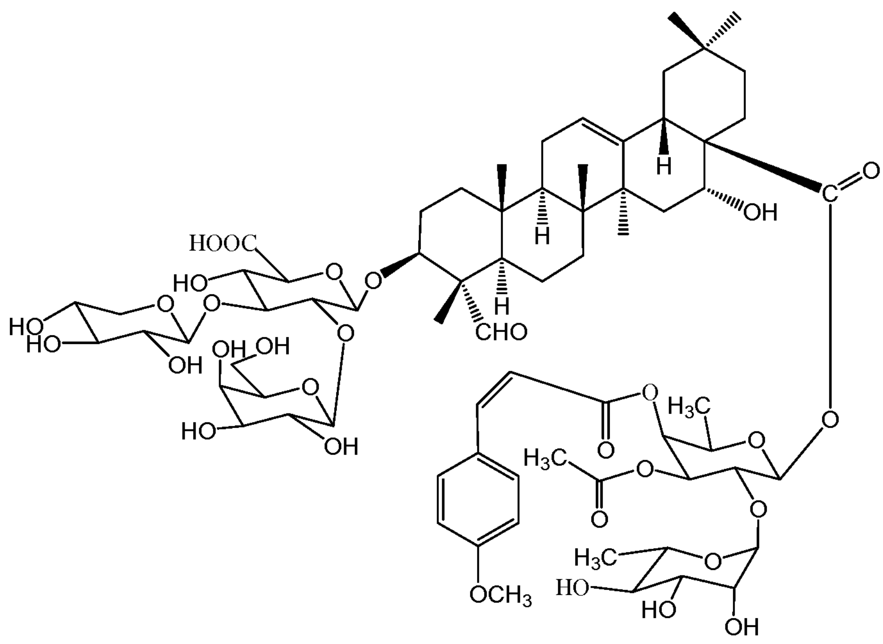

Compound (2)

Or the structural formula as follows:

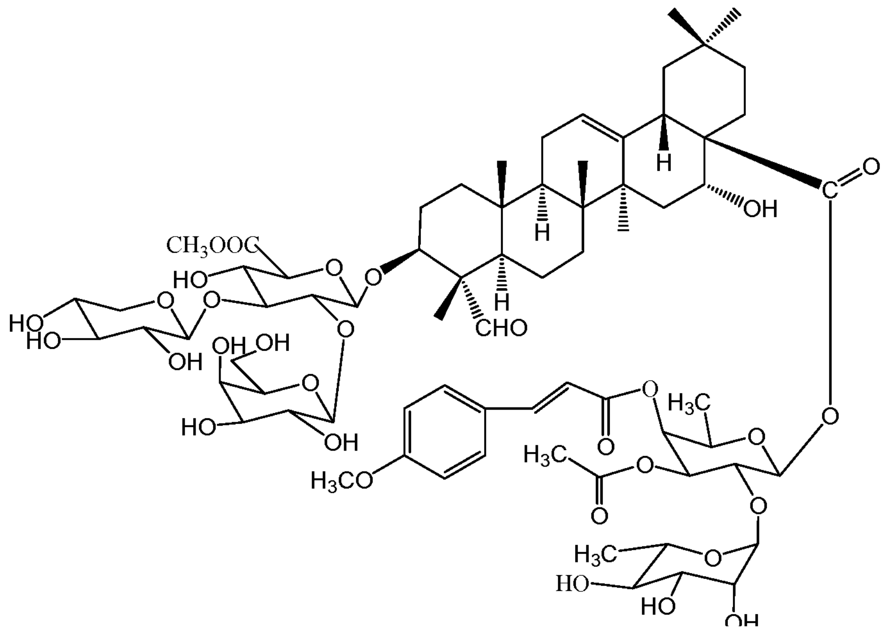

Compound (3)

Or the structural formula as follows:

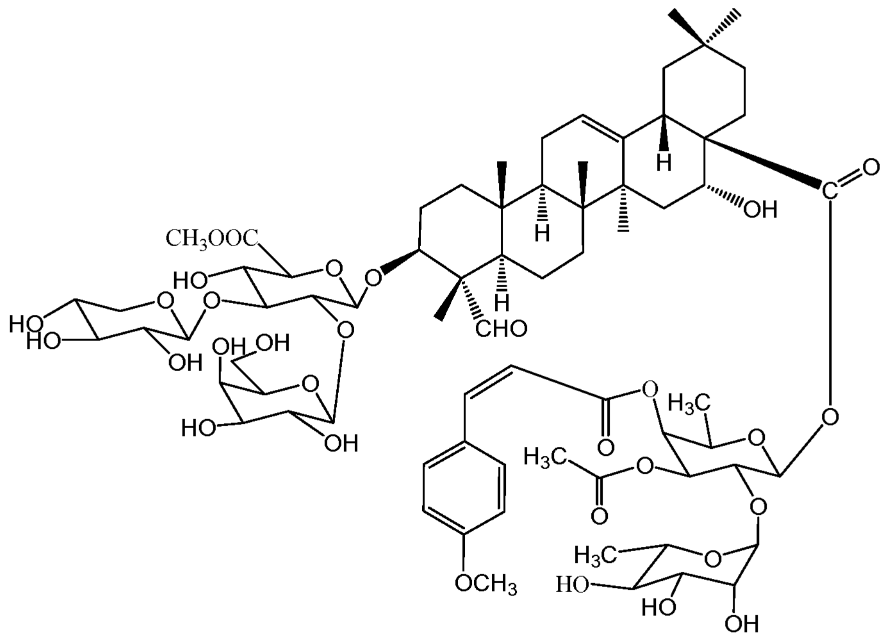

Compound (4)

Or the structural formula as follows:

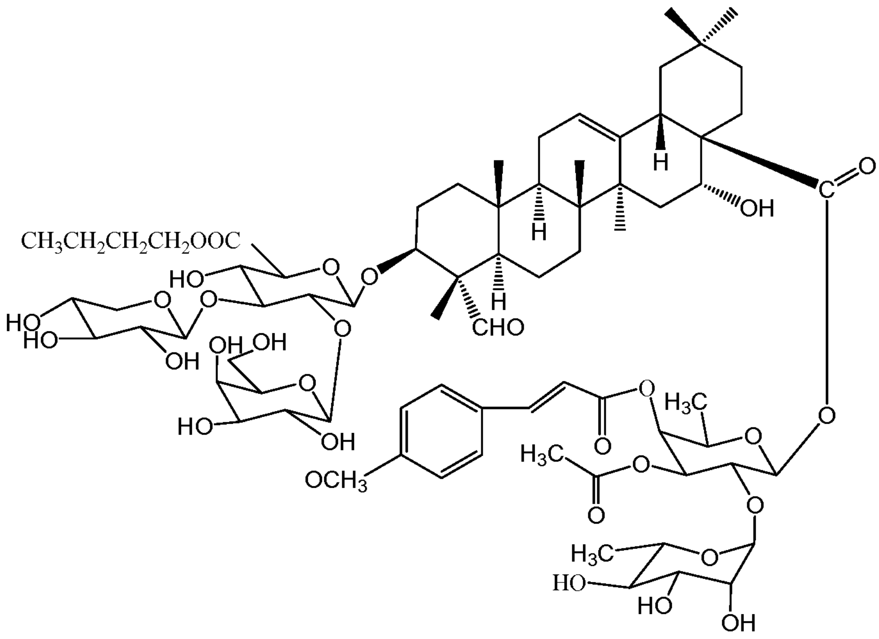

Compound (7)

Or the structural formula as follows:

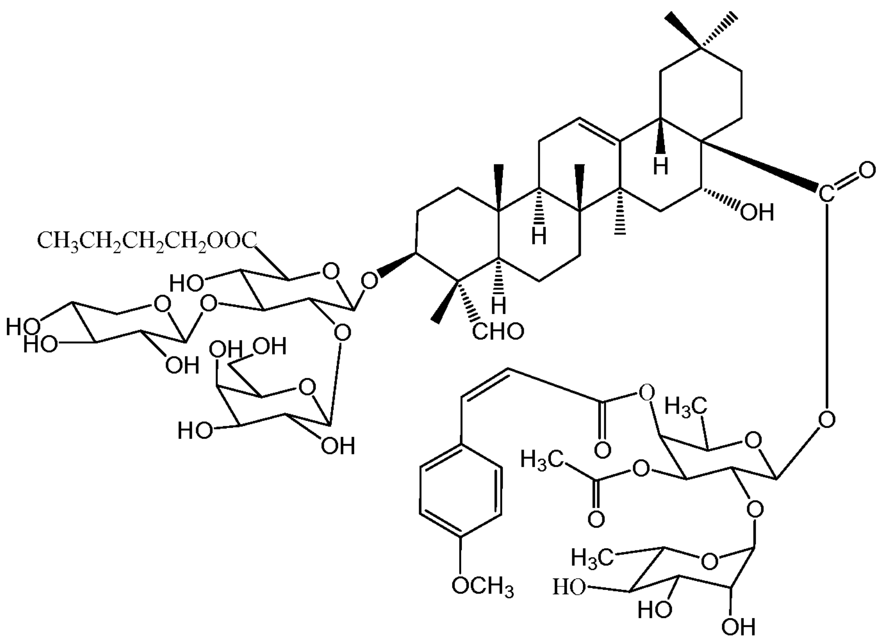

Compound (8)

11. The pharmaceutical preparation for use of claim 9, wherein the salts are compounds formed by the structural formula I and alkali or alkaline earth metal ions, optionally wherein said alkalis include sodium hydroxide, potassium hydroxide, calcium hydroxide, magnesium hydroxide, sodium bicarbonate or ammonia; the said alkaline earth metal ions include sodium, potassium, calcium, aluminum, copper, zinc and magnesium.

12. The pharmaceutical preparation for use of claim 9, wherein the compound is obtained by chemosynthesis, semisynthesis or biotransformation.

13. The pharmaceutical preparation for use of claim 9, wherein the pharmaceutical preparation is an injection, tablet, capsule, granule or dropping pill used in clinic.

## Patentansprüche

1. Verbindung der allgemeinen Formel I oder Salze davon, Zusammensetzungen, die die Verbindung der Formel I oder ihre Salze enthalten, zur Verwendung bei der Behandlung eines Tumors, wobei Formel I Folgendes ist:

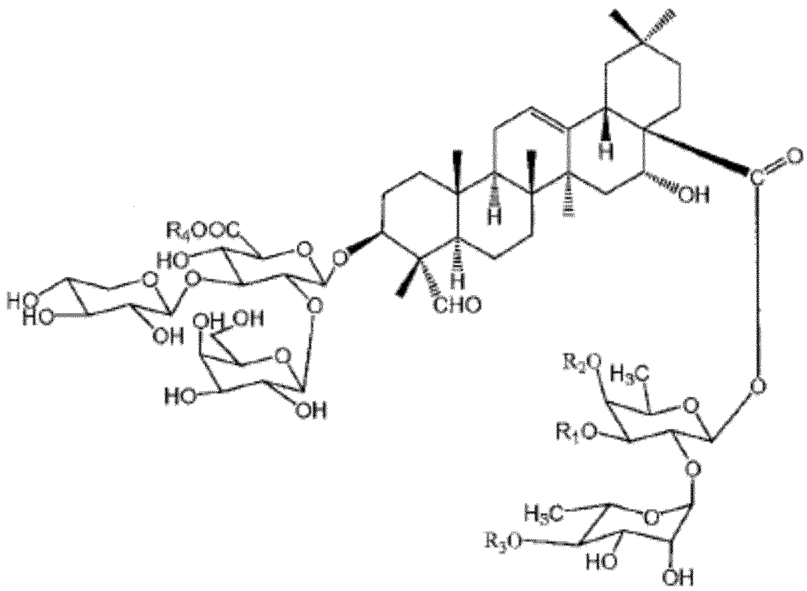

Formel I

wobei:

$R^1$ = Ac ist;
$R^2$ = (E)-MC oder (Z)-MC ist;
$R^3$ = H ist;
$R^4$ = H, $CH_3$ oder $CH_2CH_2CH_2CH_3$ ist;

wobei Ac für die folgende Gruppe steht:

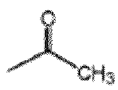

wobei (E)-MC für die folgende Gruppe steht:

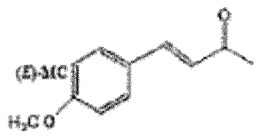

wobei (Z)-MC für die folgende Gruppe steht:

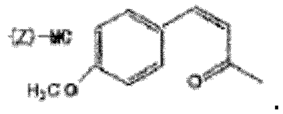

2. Verbindung zur Verwendung gemäß Anspruch 1, wobei die Verbindung I Folgendes ist:

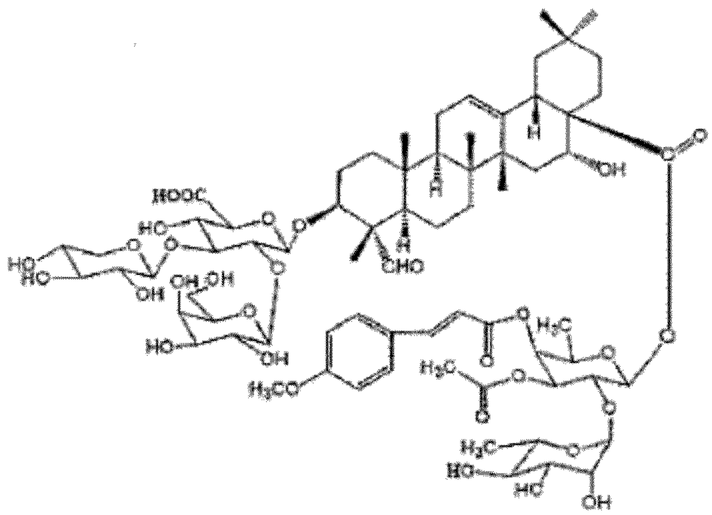

Verbindung (1)

oder die folgende Strukturformel:

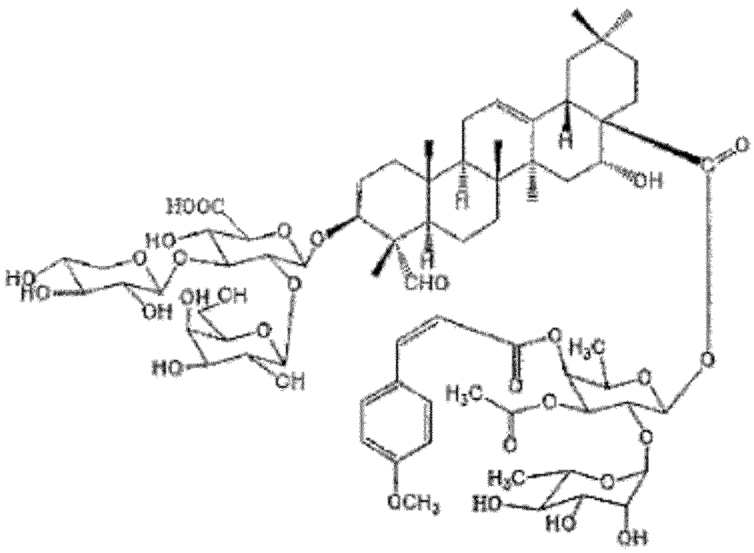

Verbindung (2)

oder die folgende Strukturformel:

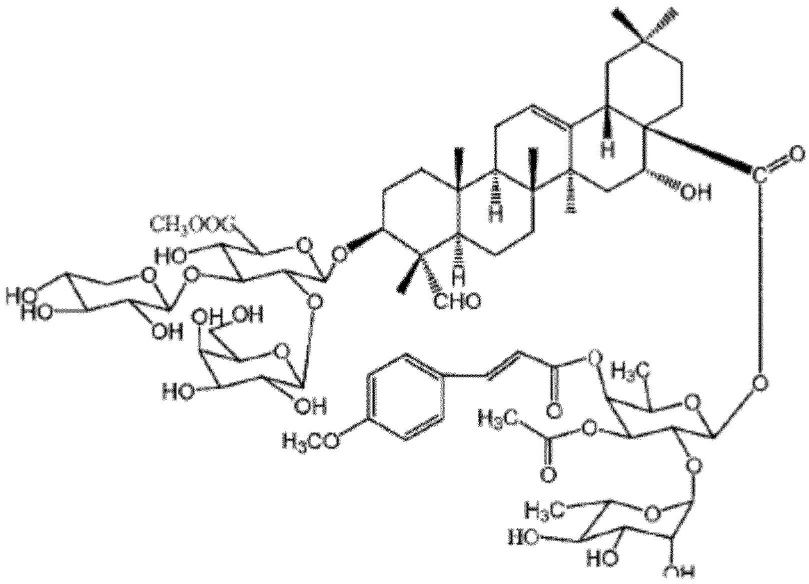

Verbindung (3)

oder die folgende Strukturformel:

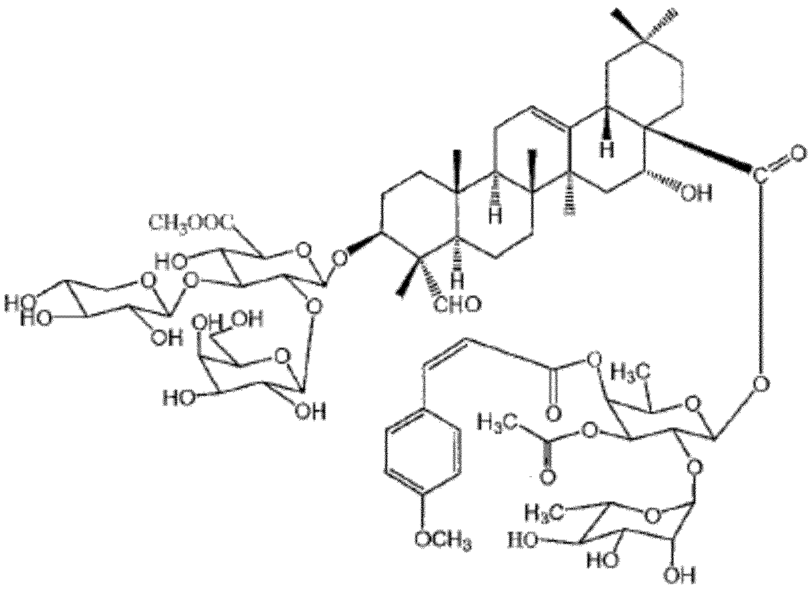

Verbindung (4)

oder die folgende Strukturformel:

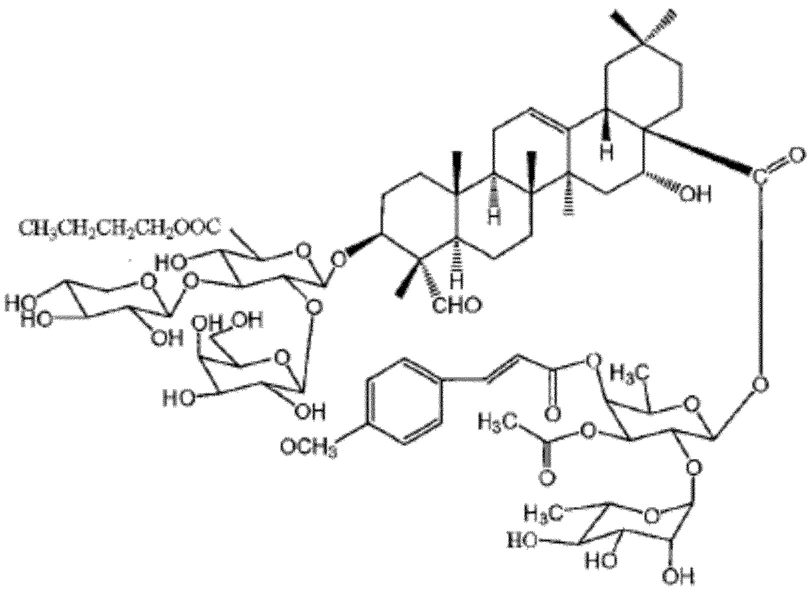

Verbindung (7)

oder die folgende Strukturformel:

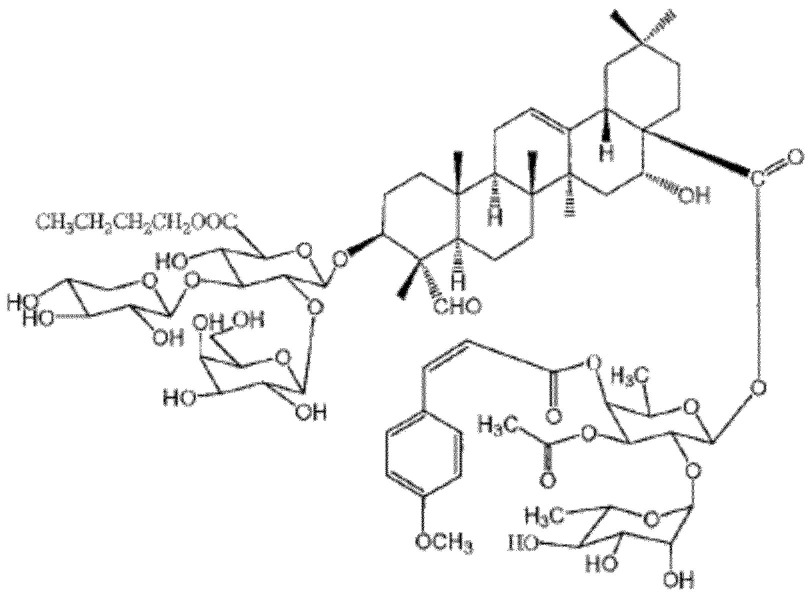

Verbindung (8).

**3.** Verbindung zur Verwendung gemäß Anspruch 1, wobei die Verbindung bei der Behandlung einer Krebserkrankung verwendet wird, die aus der Gruppe ausgewählt ist, die aus Leberkrebs, Magenkrebs, Darmkrebs, Brustkrebs und Melanom besteht.

**4.** Verbindung zur Verwendung gemäß Anspruch 1, wobei die Salze Verbindungen sind, die durch die Strukturformel I und Alkali- oder Erdalkalimetallionen gebildet werden, wobei gegebenenfalls die Alkalien Natriumhydroxid, Kaliumhydroxid, Calciumhydroxid, Magnesiumhydroxid, Natriumhydrogencarbonat oder Ammoniak umfassen, wobei die Erdalkalimetallionen Natrium, Kalium, Calcium, Aluminium, Kupfer, Zink und Magnesium umfassen.

**5.** Verbindung zur Verwendung gemäß Anspruch 1, wobei die Verbindung durch Chemosynthese, Semisynthese oder Biotransformation erhalten ist.

**6.** Pharmazeutische Zusammensetzung, die eine Verbindung der allgemeinen Formel I oder Salze davon und einen der herkömmlichen Antikrebswirkstoffe umfasst, wobei Formel I Folgendes ist:

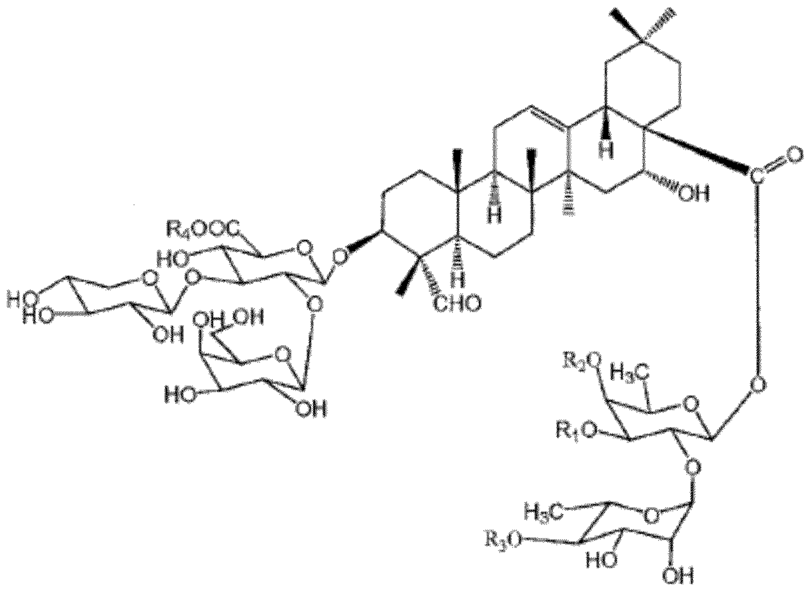

Formel I

wobei:

$R^1$ = Ac ist;
$R^2$ = (E)-MC oder (Z)-MC ist;
$R^3$ = H ist;
$R^4$ = H, $CH_3$ oder $CH_2CH_2CH_2CH_3$ ist;

wobei Ac für die folgende Gruppe steht:

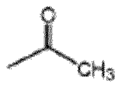

wobei (E)-MC für die folgende Gruppe steht:

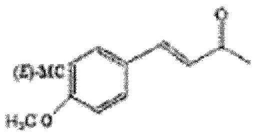

wobei (Z)-MC für die folgende Gruppe steht:

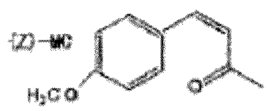

.

**7.** Pharmazeutische Zusammensetzung gemäß Anspruch 6, wobei es sich bei dem Antikrebswirkstoff um ein Cyclophosphamid oder 5-FU oder Paclitaxel handelt.

**8.** Pharmazeutische Zusammensetzung gemäß Anspruch 6 zur Verwendung bei der Behandlung eines Tumors.

**9.** Pharmazeutisches Präparat zur Verwendung bei der Behandlung eines Tumors, wobei das Präparat aus einer Verbindung der allgemeinen Formel I oder Salzen davon und Arzneimittelhilfsstoffen hergestellt ist:

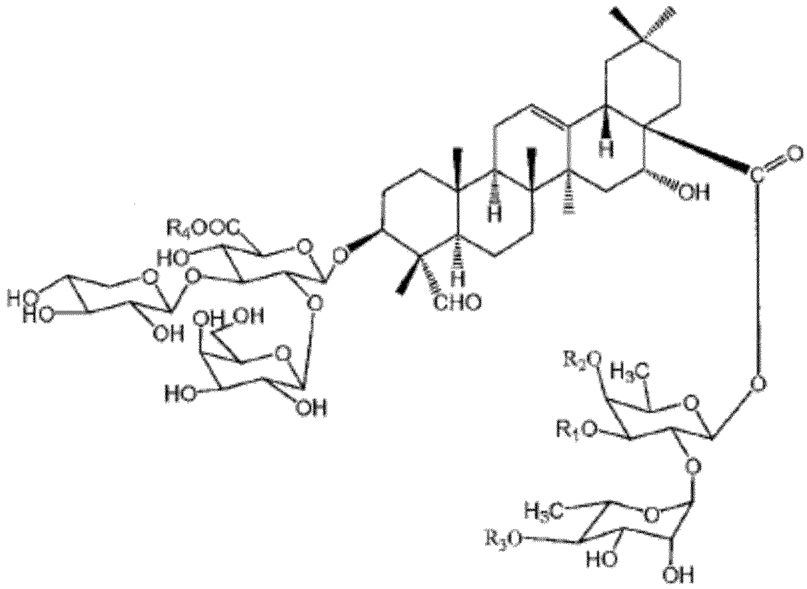

Formel I

wobei:

$R^1$ = Ac ist;
$R^2$ = (E)-MC oder (Z)-MC ist;
$R^3$ = H ist;
$R^4$ = H, $CH_3$ oder $CH_2CH_2CH_2CH_3$ ist;

wobei Ac für die folgende Gruppe steht:

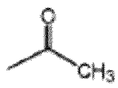

wobei (E)-MC für die folgende Gruppe steht:

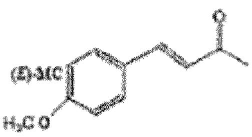

wobei (Z)-MC für die folgende Gruppe steht:

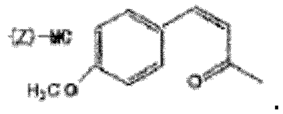

10. Pharmazeutisches Präparat zur Verwendung gemäß Anspruch 9, wobei es sich bei Verbindung (I) um die folgende Strukturformel handelt:

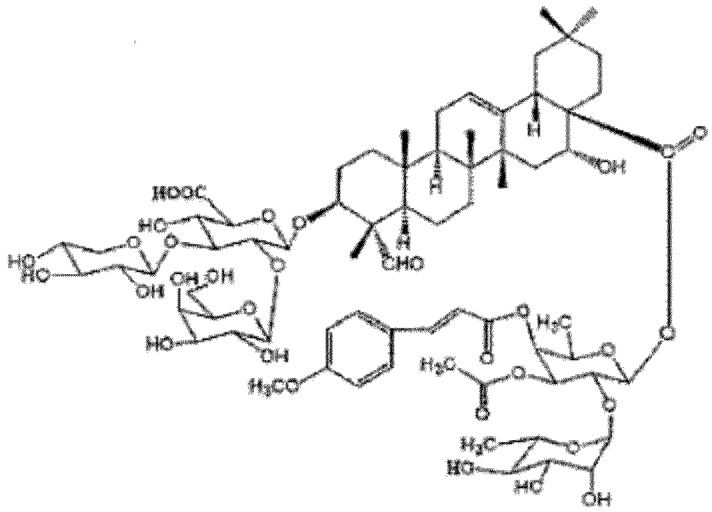

Verbindung (1)

oder die folgende Strukturformel:

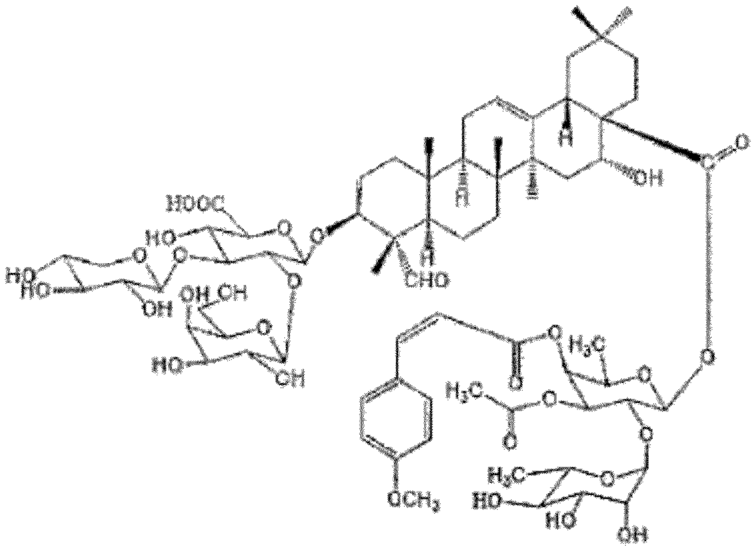

Verbindung (2)

oder die folgende Strukturformel:

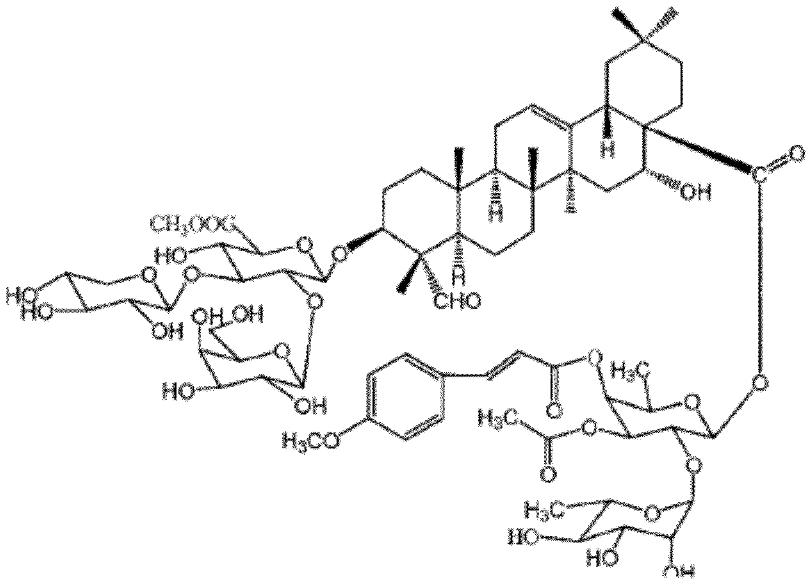

Verbindung (3)

oder die folgende Strukturformel:

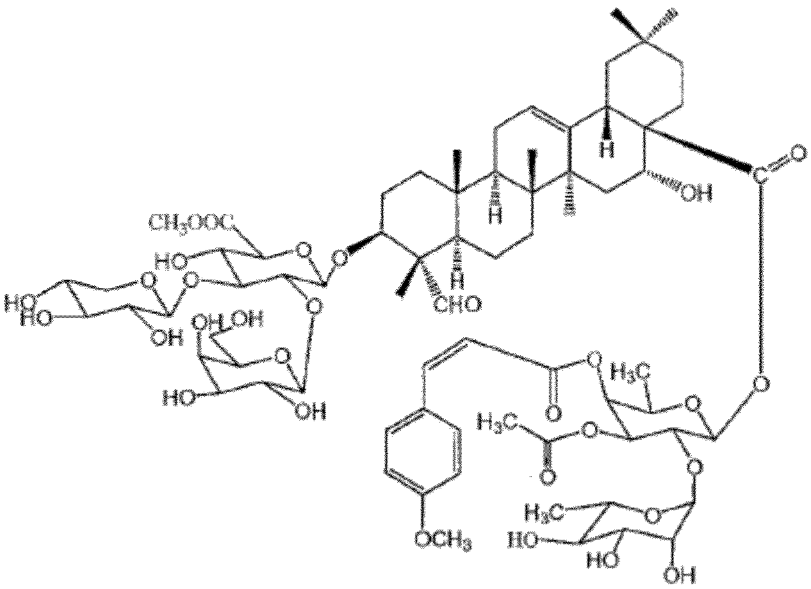

Verbindung (4)

oder die folgende Strukturformel:

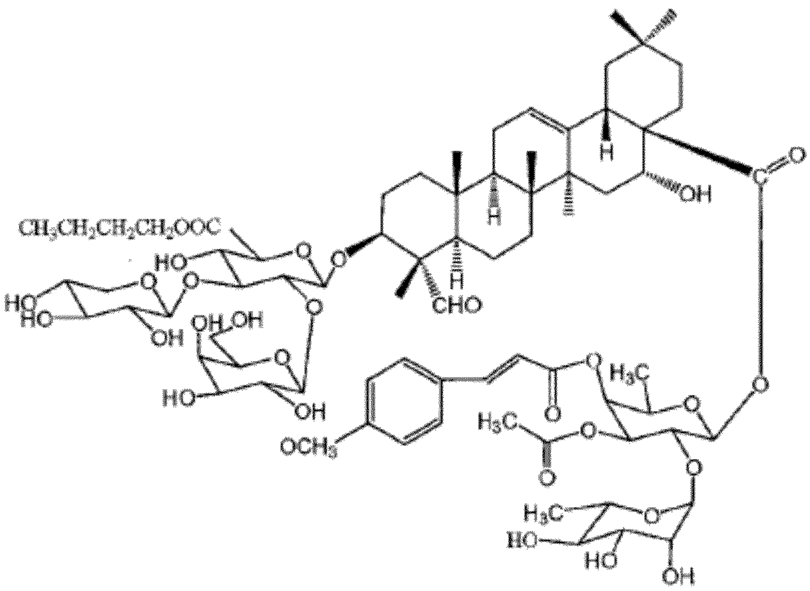

Verbindung (7)

oder die folgende Strukturformel:

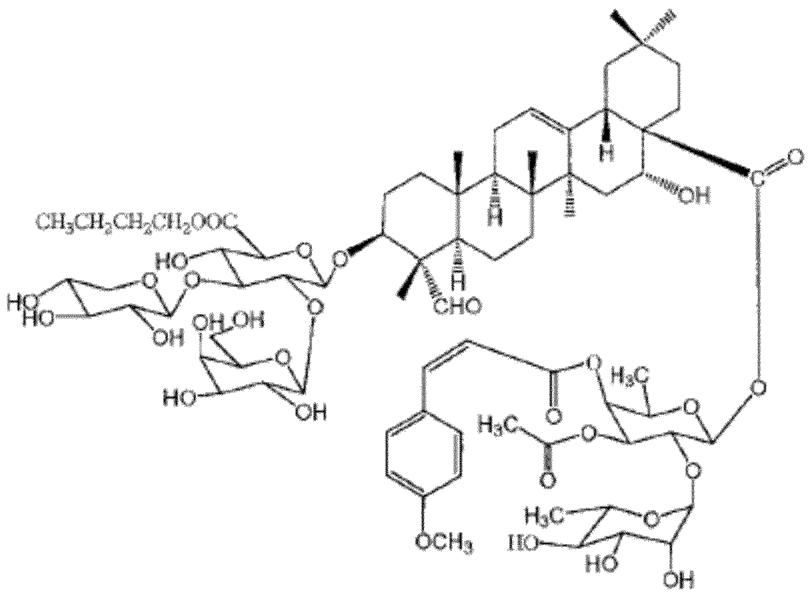

Verbindung (8).

11. Pharmazeutisches Präparat zur Verwendung gemäß Anspruch 9, wobei die Salze Verbindungen sind, die durch die Strukturformel I und Alkali- oder Erdalkalimetallionen gebildet werden, wobei gegebenenfalls die Alkalien Natriumhydroxid, Kaliumhydroxid, Calciumhydroxid, Magnesiumhydroxid, Natriumhydrogencarbonat oder Ammoniak umfassen, wobei die Erdalkalimetallionen Natrium, Kalium, Calcium, Aluminium, Kupfer, Zink und Magnesium umfassen.

12. Pharmazeutisches Präparat zur Verwendung gemäß Anspruch 9, wobei die Verbindung durch Chemosynthese, Semisynthese oder Biotransformation erhalten ist.

**13.** Pharmazeutisches Präparat zur Verwendung gemäß Anspruch 9, wobei das pharmazeutische Präparat eine Injektionslösung, Tablette, Kapsel, ein Granulat oder eine in der Klinik verwendete Tropfpille ist.

## Revendications

**1.** Composé répondant à la formule générale I ou des sels de celui-ci, compositions contenant le composé de la formule I ou ses sels, à utiliser dans le traitement d'un tumeur, dans lequel la formule I est :

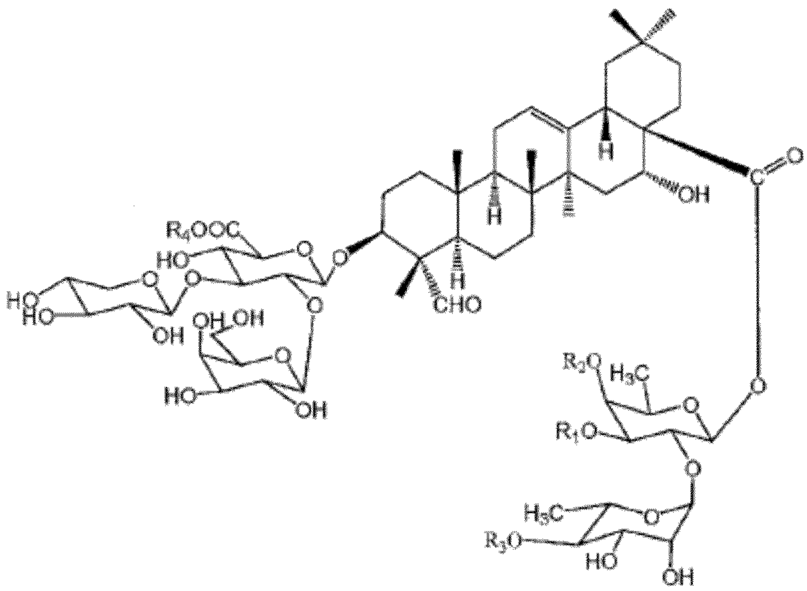

Formule I

où:

$R^1$ est Ac,
$R^2$ est (E)-MC ou (Z)-MC,
$R^3$ est H,
$R^4$ est H, $CH_3$ ou $CH_2CH_2CH_2CH_3$,

où Ac représente un groupe comme suit :

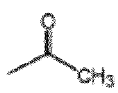

dans lequel (E)-MC représente un groupe comme suit :

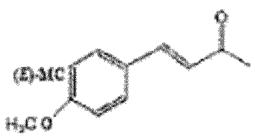

dans lequel (Z)-MC représente un groupe comme suit :

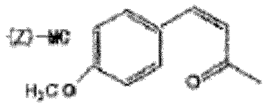

.

2. Composé à utiliser selon la revendication 1, dans lequel le composé I est :

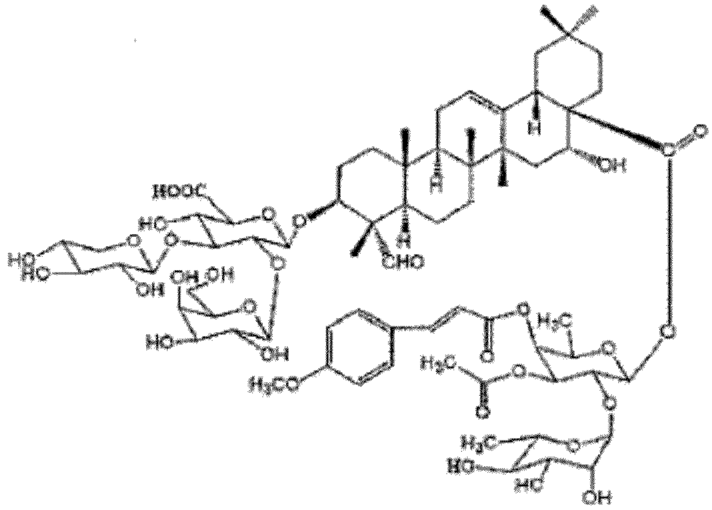

composé (1)

ou la formule structurelle suivante :

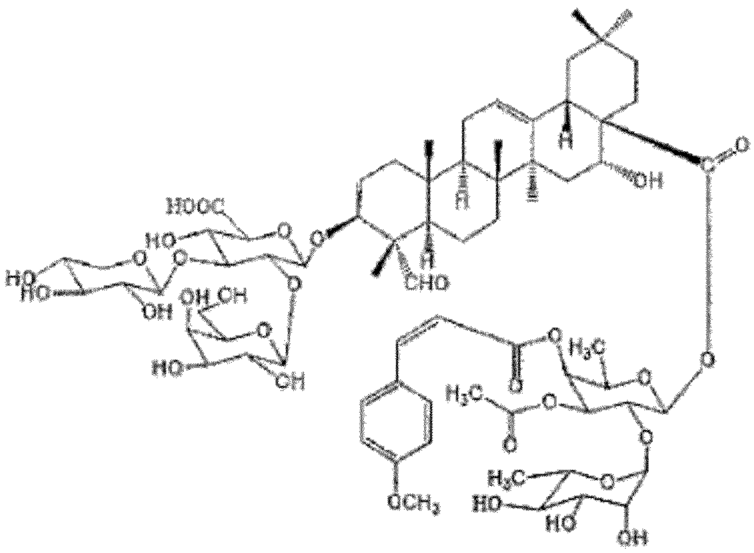

composé (2)

ou la formule structurelle :

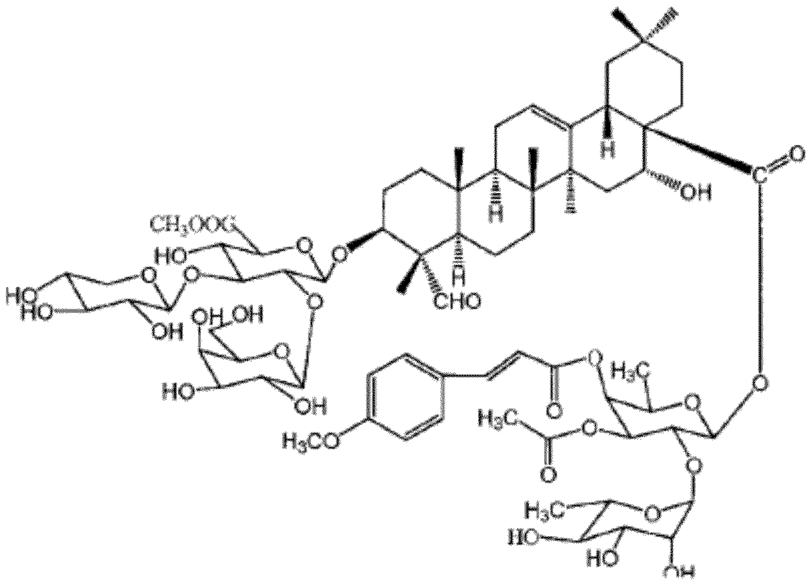

composé (3)

ou la formule structurelle :

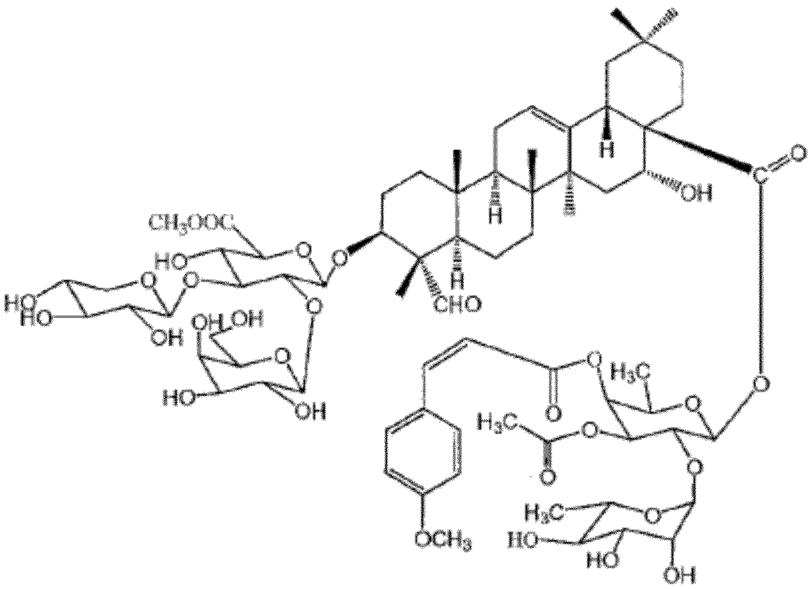

composé (4)

ou la formule structurelle :

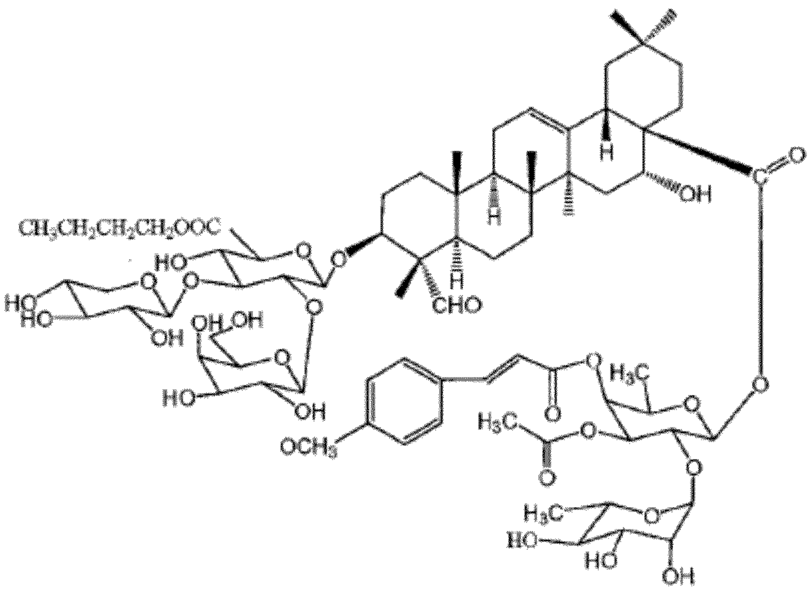

composé (7)

ou la formule structurelle :

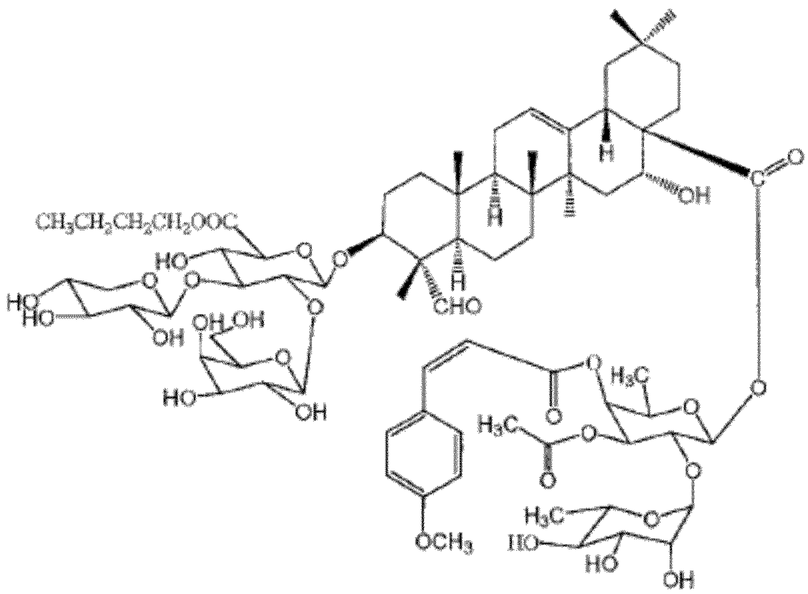

composé (8).

3. Composé à utiliser selon la revendication 1, dans lequel le composé est utilisé dans le traitement d'un cancer choisi dans le groupe consistant en cancer du foie, cancer gastrique, cancer du côlon, cancer du sein, et mélanome.

4. Composé à utiliser selon la revendication 1, dans lequel les sels sont des composés formés par la formule structurelle I et des ions de métaux alkali ou alcalino-terreux, dans lequel éventuellement lesdits alkalis comprennent l'hydroxyde de sodium, l'hydroxyde de potassium, l'hydroxyde de calcium, l'hydroxyde de magnésium, le bicarbonate de sodium, ou l'ammoniaque, lesdits ions de métaux alcalino-terreux comprennent le sodium, le potassium, le calcium, l'aluminium, le cuivre, le zinc, et le magnésium.

5. Composé à utiliser selon la revendication 1, dans lequel le composé est obtenu par chimiosynthèse, semi-synthèse, ou biotransformation.

6. Composition pharmaceutique comprenant un composé répondant à la formule générale I ou des sels de celui-ci, et l'un quelconque des agents anticancéreux, dans lequel la formule I est :

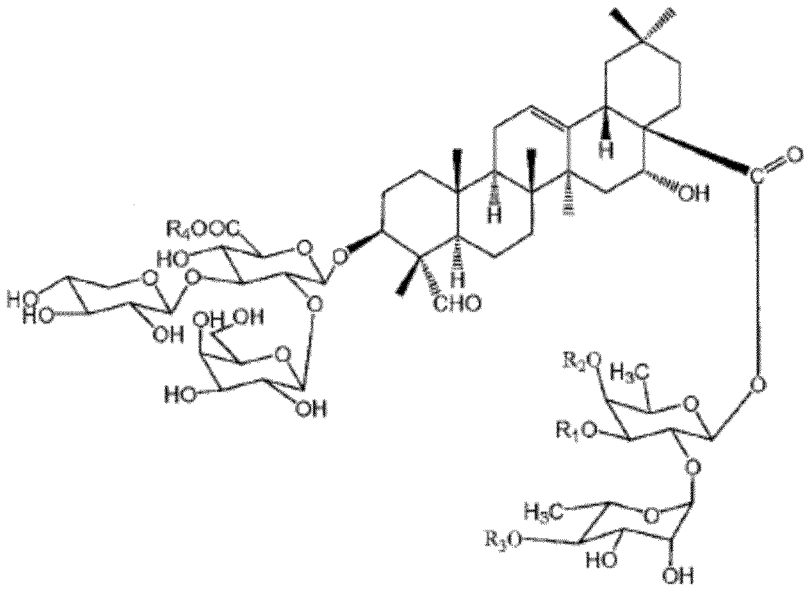

Formule I

où:

$R^1$ est Ac,
$R^2$ est (E)-MC ou (Z)-MC,
$R^3$ est H,
$R^4$ est H, $CH_3$ ou $CH2CH2CH2CH_3$,

où Ac représente un groupe comme suit :

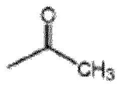

dans lequel (E)-MC représente un groupe comme suit :

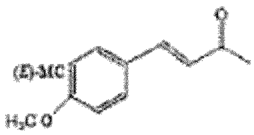

dans lequel (Z)-MC représente un groupe comme suit :

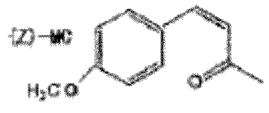.

**7.** Composition pharmaceutique selon la revendication 6, dans laquelle ledit agent anticancéreux est un cyclophosphamide ou le 5-FU ou le paclitaxel.

**8.** Composition pharmaceutique selon la revendication 6 à utiliser dans le traitement d'un tumeur.

**9.** Préparation pharmaceutique à utiliser dans le traitement d'un tumeur, dans laquelle ladite préparation est faite à partir du composé répondant à la formule générale I ou des sels de celui-ci, et des excipients :

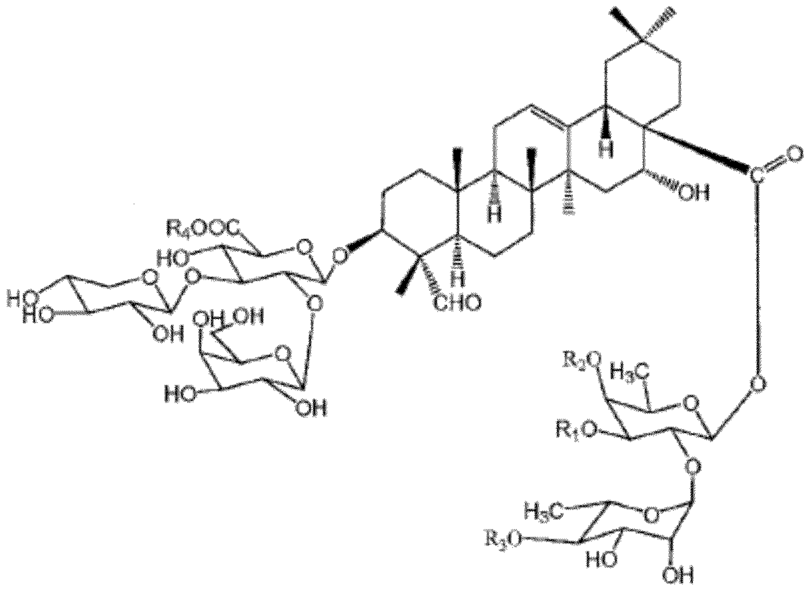

Formule I

où:

$R^1$ est Ac,
$R^2$ est (E)-MC ou (Z)-MC,
$R^3$ est H,
$R^4$ est H, $CH_3$ ou $CH2CH2CH2CH_3$,

où Ac représente un groupe comme suit :

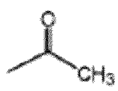

dans lequel (E)-MC représente un groupe comme suit :

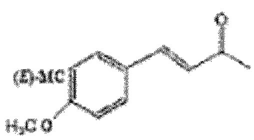

dans lequel (Z)-MC représente un groupe comme suit :

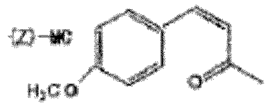

.

**10.** Préparation pharmaceutique à utiliser selon la revendication 9, dans laquelle le composé (I) répond à une formule structurelle comme suit :

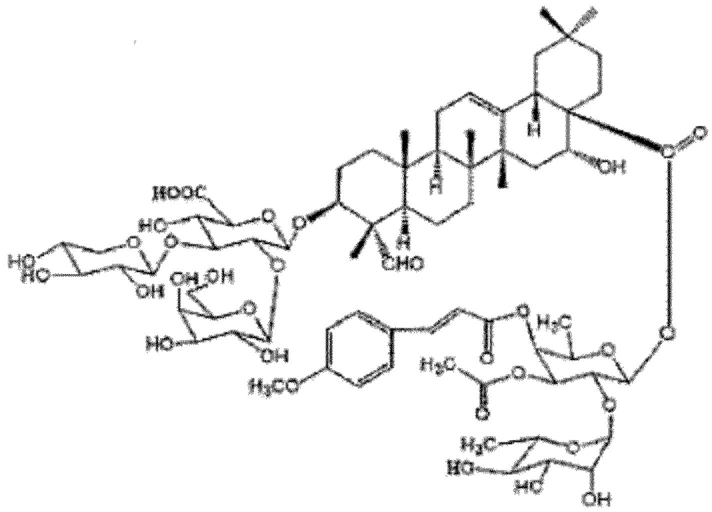

composé (1)

ou la formule structurelle suivante :

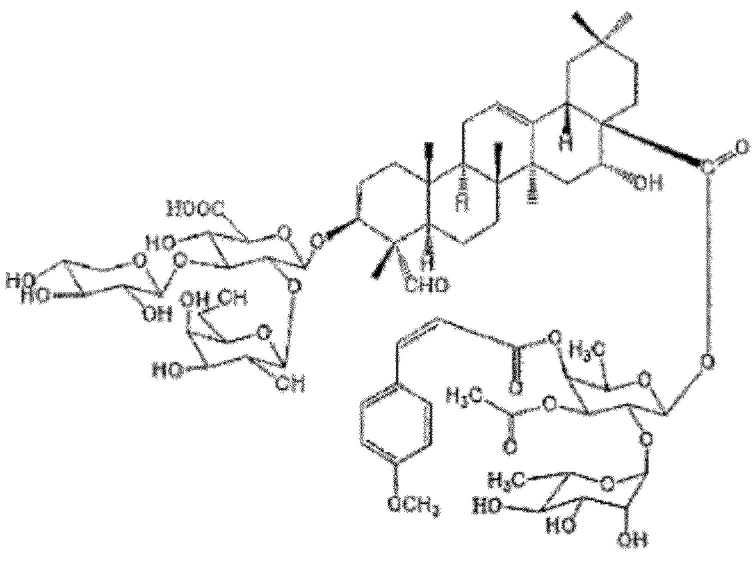

composé (2)

ou la formule structurelle :

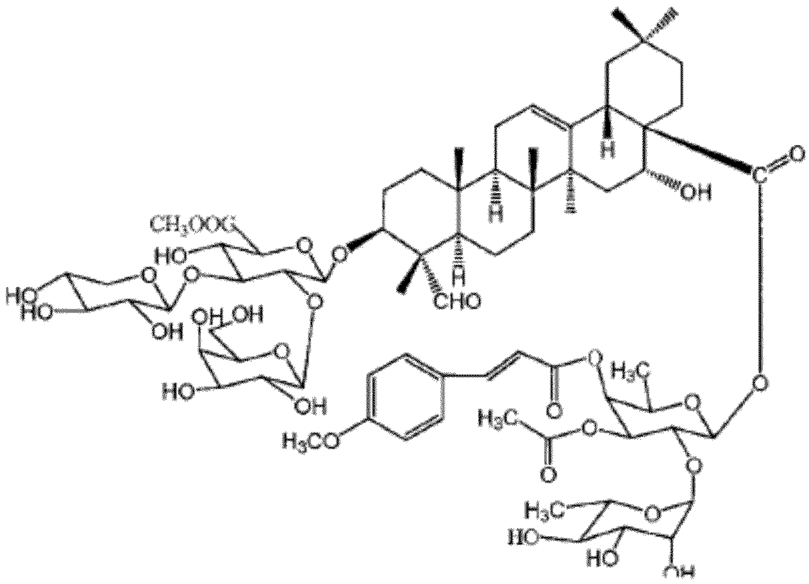

composé (3)

ou la formule structurelle :

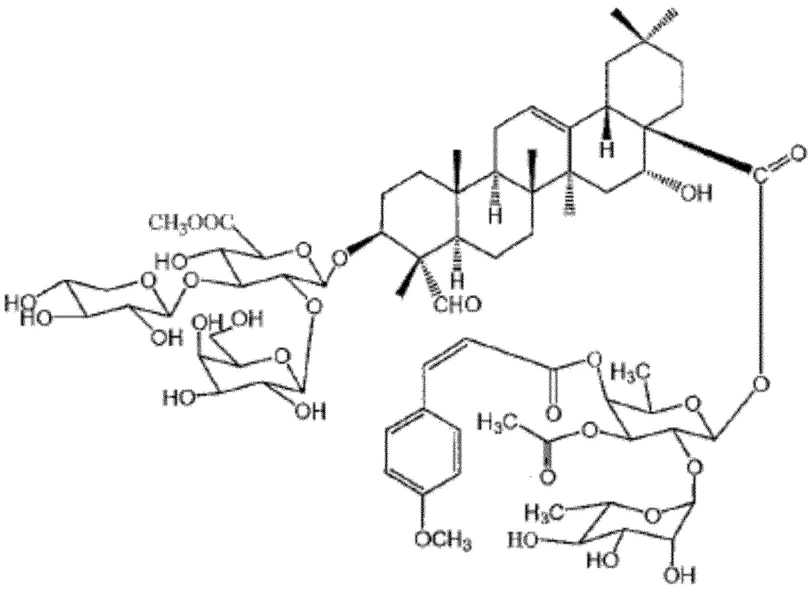

composé (4)

ou la formule structurelle :

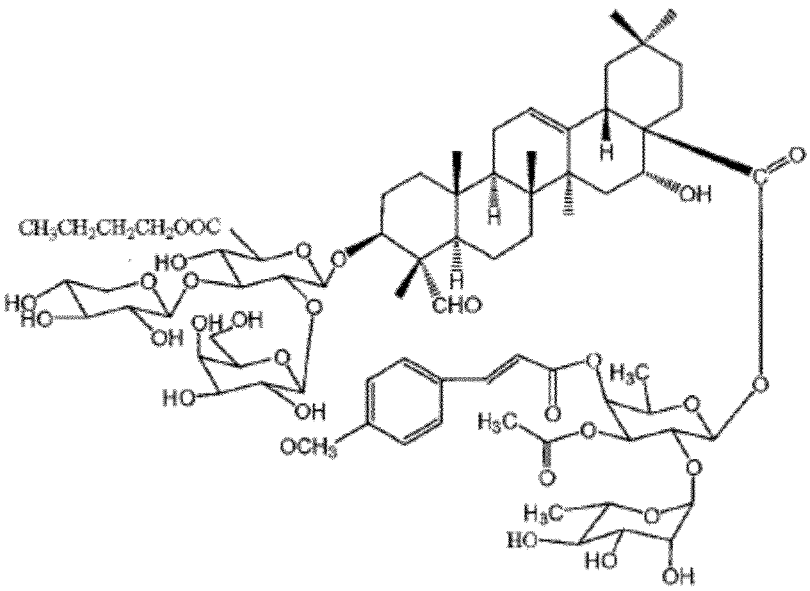

composé (7)

ou la formule structurelle :

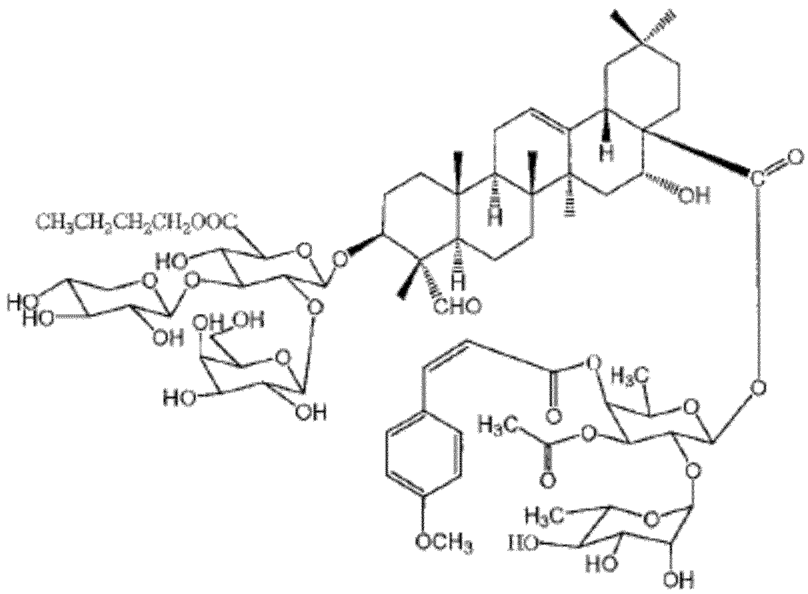

composé (8).

**11.** Préparation pharmaceutique à utiliser selon la revendication 9, dans laquelle les sels sont des composés formés par la formule structurelle I et des ions de métaux alkali ou alcalino-terreux, dans lequel éventuellement lesdits alkalis comprennent l'hydroxyde de sodium, l'hydroxyde de potassium, l'hydroxyde de calcium, l'hydroxyde de magnésium, le bicarbonate de sodium, ou l'ammoniaque, lesdits ions de métaux alcalino-terreux comprennent le sodium, le potassium, le calcium, l'aluminium, le cuivre, le zinc, et le magnésium.

**12.** Préparation pharmaceutique à utiliser selon la revendication 9, dans laquelle le composé est obtenu par chimiosynthèse, semi-synthèse, ou biotransformation.

13. Préparation pharmaceutique à utiliser selon la revendication 9, dans laquelle ladite préparation pharmaceutique est une injection, un comprimé, une capsule, un granulé, ou une pilule coulante utilisée en clinique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Isolation and Characterization of Chemical Constituents from the Roots of Silene viscidula Franch. *ASIAN JOURNAL OF CHEMISTRY,* 01 January 2013, vol. 25 (18
- **WEI XU et al.** Pentacyclic Triterpenoid Saponins from Silene viscidula. *HELVETICA CHIMICA ACTA,* 01 October 2010, vol. 93 (10), 2007-2014
- **ZHAO, J. ; NAKAMURA, N. ; HATTORI, M. ; YANG, X.W. ; KOMATSU, K. ; QIU, M.H.** New triterpenoid saponins from the roots of Sinocrassula asclepiadea. *Chemical and pharmaceutical bulletin,* 2004, vol. 52 (2), 230-237
- **BAI, H. ; ZHONG, Y. ; XIE, Y.Y. ; WANG, Y.S. ; LIU, L. ; ZHOU, L. ; WANG, J. ; MU, Y.L. ; ZUO, C.X.** A major triterpenoid saponin from Gypsophila oldhamiana. *Chemistry & biodiversity,* 2007, vol. 4 (5), 955-960